# EUROPEAN PATENT APPLICATION

(11) **EP 4 397 312 A1**
(43) Date of publication of application: **10.07.2024**
(21) Application number: 22864527.1
(22) Date of filing: 30.08.2022
(51) Int. Cl.: A61K 35/32, A61K 35/28, A61L 27/36, A61L 27/38, A61P 19/02, C12N 5/0775

(54) **THERAPEUTIC AGENT FOR ARTHROPATHY, AND METHOD FOR PRODUCING THERAPEUTIC AGENT FOR ARTHROPATHY**

(30) Priority: 31.08.2021 JP 2021140803; 27.12.2021 JP 2021212059
(71) Applicant: FUJIFILM Corporation, Tokyo 106-8620 (JP); National University Corporation Tokyo Medical and Dental University, Tokyo 113-8510 (JP)
(72) Inventor: KITAHASHI, Tsukasa, Ashigarakami-gun, Kanagawa 258-8577 (JP); KOGAWA, Ryo, Ashigarakami-gun, Kanagawa 258-8577 (JP); NAKAMURA, Kentaro, Ashigarakami-gun, Kanagawa 258-8577 (JP); SEKIYA, Ichiro, Tokyo 113-8510 (JP)
(74) Representative: Hoffmann Eitle
(86) International application number: PCT/JP2022/032502
(87) International publication number: WO 2023/032945

(57) **Abstract**

An object of the present invention is to provide a therapeutic agent for arthrosis, which contains synovium-derived mesenchymal stem cells having a molecule essential for joint medical treatment, and a production method for the therapeutic agent for arthrosis. According to the present invention, there is provided a therapeutic agent for arthrosis, which contains synovium-derived mesenchymal stem cells having any one or more kinds of surface antigens of integrin β1 or platelet-derived growth factor receptor β.

## Description

### BACKGROUND OF THE INVENTION

### Field of the Invention

The present invention relates to a therapeutic agent for arthrosis, which contains synovium-derived mesenchymal stem cells having a molecule essential for joint medical treatment, and a production method for the therapeutic agent for arthrosis.

### Description of the Related Art

In recent years, with the progress of regenerative medicine technology and cell therapy technology, various kinds of cell therapy using autologous, allogeneic, or heterologous cells, and the development of cell products for research have been actively carried out. Among the above, the mesenchymal stem cell (MSC) is expected as a cell source for the useful cell therapy. The mesenchymal stem cells can be collected from various body tissues and have been reported to be isolated from the bone marrow tissue (Non-Patent Document 1), the adipose tissue (Non-Patent Document 2), the muscle tissue (Non-Patent Document 3), the synovial tissue (Non-Patent Document 4), and the periosteal tissue (Non-Patent Document 5). In particular, it has been reported that the synovium-derived mesenchymal stem cells have a high proliferation ability and a high cartilage forming ability as compared with mesenchymal stem cells derived from various mesenchymal tissues such as bone marrow (Non-Patent Document 6). In addition, Patent Documents 1 to 3 disclose methods of medically treating articular cartilage injury and meniscus injury by using synovium-derived mesenchymal stem cells. Patent Document 4 describes a preparation method and a quality management method for a limb bud mesenchymal cell population, a cartilage progenitor cell population, and an osteogenic progenitor cell population, which use a molecule such as CD140b.

### Prior Art Documents

### Non-Patent Documents

Non-Patent Document 1: Prockop, D. J., 1997, Science. 276: 71-4
Non-Patent Document 2: Zuk, P. A. et al., 2002, Mol Biol Cell. 13: 4279-95
Non-Patent Document 3: Cao et al., 2003, Nat Cell Biol. 5: 640-6
Non-Patent Document 4: De Bari, C. et al., 2001, Arthritis Rheum. 44: 1928-42
Non-Patent Document 5: Fukumoto, T. et al., 2003, Osteoarthritis Cartilage. 11: 55-64
Non-Patent Document 6: Sakaguchi, et al., 2005, Arthritis Rhum. 52: 2521-9

### Patent Documents

Patent Document 1: JP5928961B
Patent Document 2: JP5656183B
Patent Document 3: JP6864302B
Patent Document 4: WO2021/054449A

### SUMMARY OF THE INVENTION

An object of quality management of a cell product is to ensure the equivalency and the identity of each lot of the cell product. However, since the cells themselves that constitute the product are not a perfect uniform constituent, and it is difficult to specify the characteristics thereof, it is generally difficult to ensure the equivalency and the identity of each lot of the product. Therefore, in order to manage the quality of a product, not only a quality test of the final product has been carried out but also a concept of a quality management system (QMS), which has been applied to medical devices, has been incorporated to record and control raw materials for manufacturing, material management, manufacturing process management, and a process management test, whereby the management has been carried out in terms of the entire process. However, in association with the progress in science and technology, the importance of identifying the characteristics of the cells themselves as the final product has increased.

As a quality management method for cells, it has been carried out, for example, to identify a cell type of a target final product using a cell type-specific surface marker as an indicator (for example, to identify that the cells are mesenchymal stem cells). However, there is a concern that the curing effect of cells obtained by such a quality management method is not constant and thus is not yet sufficiently satisfactory.

So far, a production method for a joint curing agent using synovial stem cells has been reported. However, there has been a problem that the quality management for ensuring the effectiveness of the therapeutic agent has not been sufficiently carried out, and the therapeutic effect is not constant. The quality management of the cell product has been mainly carried out with a marker for identifying a cell type, whereas the quality management regarding the titer (effectiveness) has not been carried out.

The marker that exhibits the titer (effectiveness) is identified based on the mechanism of action. Therefore, the present invention aims to interpret the mechanism of action involved in the effectiveness of a cell therapy product, to identify a marker based on the mechanism of action, and further to provide a production method for a therapeutic agent based on the marker. That is, an object of the present invention is to provide a therapeutic agent for arthrosis, which contains synovium-derived mesenchymal stem cells having a molecule essential for joint medical treatment, and a production method for the therapeutic agent for arthrosis.

As a result of diligent studies to achieve the above object, the inventors of the present invention have found that any one or more kinds of integrin β1 or platelet-derived growth factor receptor β are a quality management marker that is essential for the effectiveness of the medical treatment of a joint disease using synovial stem cells. The present invention has been completed based on the above findings.

That is, according to the present invention, the following inventions are provided.
<1> A therapeutic agent for arthrosis, comprising:
   synovium-derived mesenchymal stem cells having any one or more kinds of surface antigens of integrin β1 or platelet-derived growth factor receptor β.
<2> The therapeutic agent for arthrosis according to <1>, in which the synovium-derived mesenchymal stem cells have surface antigens of both the integrin β1 and the platelet-derived growth factor receptor β.
<3> The therapeutic agent for arthrosis according to <1> or <2>, in which the synovium-derived mesenchymal stem cells have a gene encoding a type II collagen α1 chain and produce the type II collagen α1 chain after being transplantation.
<4> The therapeutic agent for arthrosis according to any one of <1> to <3>, in which the synovium-derived mesenchymal stem cells have a surface antigen of FGFR3.
<5> The therapeutic agent for arthrosis according to any one of <1> to <4>, in which a ratio of the synovium-derived mesenchymal stem cells having any one or more kinds of surface antigens of integrin β1 or platelet-derived growth factor receptor β to all cells contained in the therapeutic agent for arthrosis is 30% or more.
<6> A production method for the therapeutic agent for arthrosis according to any one of <1> to <5>, comprising:
   a step A of treating a synovial tissue with an enzyme;
   a step B of washing an enzyme-treated mixture;
   a step C of culturing synovium-derived mesenchymal stem cells, which are contained in a washed mixture, on a base material; and
   a step D of separating cultured synovium-derived mesenchymal stem cells from the base material.
<7> The method according to <6>, in which the step B is a step of washing the enzyme-treated mixture until a residual enzyme concentration in a supernatant is 0.5 ng/mL or less.
<8> The method according to <6> or <7>, in which in the step C, a period during which the synovium-derived mesenchymal stem cells are cultured is 28 days or less.
<9> The method according to any one of <6> to <8>, in which in the step D, the mesenchymal stem cells are separated by allowing a cell stripper to act on the mesenchymal stem cells for a time within 120 minutes.
<10> The method according to any one of <6> to <9>, further comprising:
   a step of sorting out synovium-derived mesenchymal stem cells having any one or more kinds of surface antigens of integrin β1 or platelet-derived growth factor receptor β.

The therapeutic agent for arthrosis according to the aspect of the present invention contains synovium-derived mesenchymal stem cells having any one or more kinds of surface antigens of integrin β1 or platelet-derived growth factor receptor β, thereby being capable of exhibiting a therapeutic effect on arthrosis. According to the present invention, it is possible to suppress the fluctuation of a therapeutic effect of a produced therapeutic agent for arthrosis, and it is possible to carry out quality management for a therapeutic effect of a product.

### BRIEF DESCRIPTION OF THE DRAWINGS

Fig. 1 shows the results obtained by investigating the suppression of the extracellular matrix adhesion ability of rat synovium-derived mesenchymal stem cells by the inhibition of integrin β1.
Fig. 2 shows the results obtained by investigating the suppression of the cell proliferation ability of rat synovium-derived mesenchymal stem cells by the inhibition of PDGFRb.
Fig. 3 shows a base sequence (first half) of Col2A1 for a wild type (Col2A1WT-rSMSC) and a deletion type (Col2A1KO-rSMSC) of a Col2A1 gene of a rat synovial stem cell.
Fig. 4 shows a base sequence (second half) of Col2A1 for a wild type (Col2A1WT-rSMSC) and a deletion type (Col2A1KO-rSMSC) of the Col2A1 gene of the rat synovial stem cell.
Fig. 5 shows a base sequence (first half) of Col2A1 for a wild type (Col2A1WT-rSMSC) and a deletion type (Col2A1KO-rSMSC) of the Col2A1 gene of the rat synovial stem cell.
Fig. 6 shows a base sequence (second half) of Col2A1 for a wild type (Col2A1WT-rSMSC) and a deletion type (Col2A1KO-rSMSC) of the Col2A1 gene of the rat synovial stem cell.
Fig. 7 shows an amino acid sequence that is translated based on the base sequence of Col2A1 for the wild type (Col2A1WT-rSMSC) of the Col2A1 gene of the rat synovial stem cell.
Fig. 8 shows an amino acid sequence that is translated based on the base sequence of Col2A1 for the deletion type (Col2A1KO-rSMSC) of the Col2A1 gene of the rat synovial stem cell.
Fig. 9 shows an amino acid sequence that is translated based on the base sequence of Col2A1 for the deletion type (Col2A1KO-rSMSC) of the Col2A1 gene of the rat synovial stem cell.
Fig. 10 shows a base sequence of CD120a for a wild type (CD120aWT-rSMSC) and a deletion type (CD120aKO-rSMSC) of a CD120a gene of the rat synovial stem cell.
Fig. 11 shows an amino acid sequence that is translated based on the base sequence of CD120a for the wild type (CD120aWT-rSMSC) and the deletion type (CD120aKO-rSMSC) of the CD 120a gene of the rat synovial stem cell.
Fig. 12 shows a base sequence of CD106 for a wild type (CD106WT-rSMSC) and a deletion type (CD106KO-rSMSC) of a CD 106 gene of the rat synovial stem cell.
Fig. 13 shows an amino acid sequence that is translated based on the base sequence of CD106 for the wild type (CD106WT-rSMSC) and the deletion type (CD106KO-rSMSC) of the CD 106 gene of the rat synovial stem cell.
Fig. 14 shows the results obtained by investigating the suppression of the cartilage differentiation potency of rat synovium-derived mesenchymal stem cells from which Col2A1 has been deleted.
Fig. 15 shows the results obtained by investigating the suppression of the cartilage differentiation potency of rat synovium-derived mesenchymal stem cells from which CD120a has been deleted.
Fig. 16 shows the results obtained by investigating the suppression of the cartilage differentiation potency of rat synovium-derived mesenchymal stem cells from which CD106 has been deleted.
Fig. 17 shows the results obtained by checking the effect of regenerating the meniscus due to rat synovium-derived mesenchymal stem cells in which integrin β1 has been inhibited.
Fig. 18 shows the results obtained by checking the effect of regenerating the meniscus due to rat synovium-derived mesenchymal stem cells in which PDGFRb has been inhibited.
Fig. 19 shows the results obtained by checking the effect of regenerating the meniscus due to rat synovium-derived mesenchymal stem cells in which CD44 has been inhibited.
Fig. 20 shows the results obtained by checking the effect of regenerating the meniscus due to rat synovium-derived mesenchymal stem cells from which Col2A1 (Col2A1KO-rSMSC) has been deleted.
Fig. 21 shows the results obtained by checking the effect of regenerating the meniscus due to rat synovium-derived mesenchymal stem cells from which CD120a (CD120aKO-rSMSC) has been deleted.
Fig. 22 shows the results obtained by checking the effect of regenerating the meniscus due to rat synovium-derived mesenchymal stem cells from which CD106 (CD106KO-rSMSC) has been deleted.
Fig. 23 shows the results obtained by checking the effect of regenerating the meniscus due to rat synovium-derived mesenchymal stem cells in which FGFR3 has been inhibited.

### DESCRIPTION OF THE PREFERRED EMBODIMENTS

Hereinafter, the contents of the present invention will be described in detail. It is noted that in the present specification, "to" is used to mean that numerical values described before and after "to" are included as a lower limit value and an upper limit value, respectively.

The therapeutic agent for arthrosis according to the embodiment of the present invention contains synovium-derived mesenchymal stem cells having any one or more kinds of surface antigens of integrin β1 or platelet-derived growth factor receptor β (also referred to as PDGFRb in the present specification).

The synovium-derived mesenchymal stem cell may have only one of integrin β1 or platelet-derived growth factor receptor β; however, it preferably has both integrin β1 and platelet-derived growth factor receptor β.

It is preferable that the synovium-derived mesenchymal stem cells have a gene encoding a type II collagen α1 chain and produce the type II collagen α1 chain after being transplanted, whereby a therapeutic effect can be exhibited.

The synovium-derived mesenchymal stem cell preferably has a surface antigen of fibroblast growth factor receptor 3 (FGFR3).

The ratio of the synovium-derived mesenchymal stem cells having any one or more kinds of surface antigens of integrin β1 or platelet-derived growth factor receptor β to all cells contained in the therapeutic agent for arthrosis according to the embodiment of the present invention is preferably 30%, and it may be 40% or more, 50% or more, 60% or more, 70% or more, 80% or more, 90% or more, or 95% or more.

The therapeutic agent for arthrosis according to the embodiment of the present invention can be produced by using a method including:
a step A of treating a synovial tissue with an enzyme;
a step B of washing an enzyme-treated mixture;
a step C of culturing synovium-derived mesenchymal stem cells, which are contained in a washed mixture, on a base material; and
a step D of separating cultured synovium-derived mesenchymal stem cells from the base material.

### <Step A of treating synovial tissue with enzyme>

The synovial tissue can be collected from the unloaded portion of the joint under anesthesia.

The biological origin of the synovial tissue is not particularly limited, and a synovial tissue derived from any biological material, preferably a mammal, can be used. For example, a synovial tissue derived from a primate (for example, a chimpanzee, a Japanese monkey, or a human) can be used, and a human-derived synovial tissue can be used particularly preferably.

The synovial tissue may be a synovial tissue derived from a single donor or may be a synovial tissue derived from a plurality of donors; however, it is preferably a synovial tissue derived from a single donor.

In a case of producing synovium-derived mesenchymal stem cells for the intended purpose of administration to a human, it is preferable to use a synovial tissue collected from a donor of which histocompatibility antigen type is identical or similar to that of the recipient. More preferably, a subject from which the synovium is collected and a subject to which the synovium-derived mesenchymal stem cell is transplanted are the same subject. That is, it is preferable to use the synovial tissue collected from the recipient itself (autologous transplantation).

The amount of the synovial tissue to be collected can be determined in consideration of the type of donor or the required amount of synovium-derived mesenchymal stem cells. For example, it is possible to obtain synovium-derived mesenchymal stem cells from the synovial tissue of 0.1 g to 10 g, preferably 0.1 g to 2.0 g, more preferably 0.1 g to 1.5 g, and still more preferably 0.1 g to 1.0 g. The collected synovial tissue is shredded with scissors or the like as necessary, and then subjected to the enzyme treatment described below.

The synovial tissue is treated with an enzyme.

The enzyme is not particularly limited as long as it is an enzyme including a protease. However, it is preferably a mixed enzyme including one or more kinds of collagenases and one or more kinds of neutral proteases. A particularly preferred enzyme is Liberase (registered trade name). As the Liberase (registered trade name), it is possible to use, for example, Liberase MNP-S (manufactured by F. Hoffmann-La Roche, Ltd.), which is an enzyme including a collagenase class I, a collagenase class II, and a neutral protease (thermolysin).

The enzymatic reaction can be carried out in an aqueous solution containing an enzyme, and an aqueous solution containing human serum may be used. The human serum may be an autologous serum or an allogeneic serum; however, it is preferably an autologous serum.

The enzyme concentration in the enzyme treatment is preferably 0.01 mg/ml to 10 mg/ml, more preferably 0.1 mg/ml to 10 mg/ml, still more preferably 0.5 mg/ml to 10 mg/ml, ever still more preferably 0.5 mg/ml to 5.0 mg/ml, particularly preferably 0.5 mg/ml to 2.0 mg/ml, and most preferably 0.7 mg/ml to 2.0 mg/ml.

The mass ratio of the synovial tissue to the enzyme is preferably 1,000:1 to 10:1, more preferably 500: 1 to 20:1, and still more preferably 200:1 to 40:1.

The enzymatic reaction can be carried out at a temperature of preferably 15°C to 40°C, more preferably 20°C to 35°C, and still more preferably 25°C to 35°C.

The reaction time may be 2 hours or more, more preferably 2.5 hours or more, and still more preferably 3 hours or more. The upper limit of the reaction time is not particularly limited; however, it may be 10 hours or less, 9 hours or less, 8 hours or less, 7 hours or less, 6 hours or less, 5 hours or less, or 4 hours or less.

The enzyme-treated mixture contains synovium-derived mesenchymal stem cells.

The enzyme-treated mixture is transferred to a centrifuge tube through a cell strainer and centrifuged, whereby synovium-derived mesenchymal stem cells can be recovered.

### <Step B of washing enzyme-treated mixture>

In the step B, an enzyme-treated mixture is washed.

In the step B, it is preferable to carry out washing until the residual enzyme concentration in the supernatant is 0.5 ng/mL or less. The residual enzyme concentration in the supernatant is more preferably 0.3 ng/mL or less, still more preferably 0.2 ng/mL or less, and particularly preferably 0.1 ng/mL or less.

The washing can be carried out by resuspending the synovium-derived mesenchymal stem cells recovered with the above-described centrifugation treatment in a culture medium and carrying out centrifugation again (for example, at 400 g for 5 minutes). As the culture medium, α-modified Eagle minimum essential medium (αMEM) can be used, which is not particularly limited. The washing may be carried out a plurality of times (two times or more) using the culture medium as described above.

### <Step C of culturing, on base material, synovium-derived mesenchymal stem cells, which are contained in washed mixture>

In the step C, synovium-derived mesenchymal stem cells, which are contained in a washed mixture, are cultured on a base material.

Examples of the base material include a flat plastic base material such as a culture plate and a three-dimensional base material such as a culture bag, a microcarrier, or a gel, which are not particularly limited.

As the culture medium that is used in the culture, a culture medium that is used for the general culture of animal cells can be prepared as a basal medium. Examples of the culture medium that is used for general culture of animal cells include αMEM, a Dulbecco's modified Eagle medium (DMEM), a mixed medium of DMEM and F12 (DMEM:F12 = 1:1), an RPMI medium (a GIBCO (registered trade name) RPMI 1640 medium or the like), and a mixed medium of DMEM/F12 and RPMI (DMEM/F12:RPMI = 1:1), which are not particularly limited.

The culture medium may be a culture medium containing serum or may be a culture medium not containing serum. In a case of producing synovium-derived mesenchymal stem cells from the autologous tissue for the intended purpose of administration to a living body, the culture medium may contain a serum of origin of the same species. That is, in a case of producing synovium-derived mesenchymal stem cells from a human tissue for the intended purpose of administration to a human, a culture medium containing a human serum may be used. In a case where serum is used, the serum may be an autologous serum or may be an allogeneic serum; however, it is preferably an autologous serum. In a case where serum is used, the adding amount of serum in the culture medium is, for example, 20% by volume or less, 10% by volume or less, or 5% by volume or less.

The cell culture conditions are not particularly limited, and general cell culture conditions can be employed. Examples thereof include culture at a temperature of 30°C to 40°C and 3% to 7% CO₂, which are not particularly limited. One example thereof includes culture at a temperature of 37°C and 5% CO₂.

In the present invention, it is preferable to carry out culture without culture medium exchange. In addition, in the above-described culturing, it is preferable that the synovium-derived mesenchymal stem cells are produced without being co-cultured with cells other than the synovium-derived mesenchymal stem cells.

The differentiation of synovium-derived mesenchymal stem cells into cartilage cells progresses as the culture period increases, and thus, it is known that the in situ cartilage forming ability of synovium-derived mesenchymal stem cells decreases when the culture period exceeds a specific length. Therefore, in the present invention, it is preferable to adjust the culture period in order to proliferate the synovium-derived mesenchymal stem cells in an undifferentiated state and in a state of having a good in situ cartilage forming ability. In the step C, it is preferable that a period during which the synovium-derived mesenchymal stem cells are cultured is 28 days or less.

In addition, in the present invention, it is necessary to consider the need to prepare a sufficient number of undifferentiated synovial stem cells in order to cover the cartilage injury part and regenerate the affected part. As a result, the culture period is preferably 5 days or more, 7 days or more, or 10 days or more, more preferably 10 to 14 days, 10 to 21 days, or 10 to 28 days, and still more preferably 10 to 21 days.

It is known that mesenchymal stem cells are differentiated into cartilage cells by culturing them in a cartilage forming culture medium to which transforming growth factor β3 (TGF-β3), dexamethasone, and bone morphogenetic protein 2 (BMP-2) have been added, whereby the cartilage tissue can be prepared in vitro. As a result, in the present invention, the synovium-derived mesenchymal stem cells isolated are preferably cultured in the absence of TGF-β3, dexamethasone, or BMP-2 in order to prevent the synovium-derived mesenchymal stem cells from being differentiated into cartilage cells.

It is also known that in the synovium-derived mesenchymal stem cells, the in situ cartilage forming ability decreases in inverse proportion to the number of passages of the mesenchymal stem cells in vitro. As a result, it is preferable to produce synovium-derived mesenchymal stem cells in the primary culture or first subculture in order to prepare undifferentiated mesenchymal stem cells,

In the present invention, the serum that is used in an autologous medical treatment is of autologous origin, the amount of serum that can be collected from the donor in an autologous medical treatment is limited, and the cell density is required to be equal to or higher than a certain level from the viewpoint of the proliferation of synovium-derived mesenchymal stem cells. For these reasons, it is preferable that the synovium-derived mesenchymal stem cells after the enzyme treatment are seeded at a cell density of 100 cells/cm² or more and 5,000 cells/cm² or less, 200 cells/cm² or more and 5,000 cells/cm² or less, 500 cells/cm² or more and 5,000 cells/cm² or less, 500 cells/cm² or more and 2,500 cells/cm² or less, or 500 cells/cm² or more and 2,000 cells/cm² or less, and then cultured. In addition, in order to proliferate the synovium-derived mesenchymal stem cells after the enzyme treatment, it is more preferable to culture the cells for 10 days or more.

The number of cells obtained at the end of the culture is preferably 1.0 × 10⁷ cells or more, 2.0 × 10⁷ cells or more, 2.5 × 10⁷ cells or more, or 3.0 × 10⁷ cells or more, more preferably 4.0 × 10⁷ cells or more, still more preferably 5.0 × 10⁷ cells or more, and particularly preferably 6.0 × 10⁷ cells or more.

### <Step D of separating cultured synovium-derived mesenchymal stem cells from base material>

In the step D, the cultured synovium-derived mesenchymal stem cells are separated from the base material. In the step D, preferably, the mesenchymal stem cells can be separated by allowing a cell stripper to act on the mesenchymal stem cells for a time within 120 minutes. The cell stripper is a solution containing a trypsin-like enzyme and EDTA. A particularly preferred enzyme is TrypLE. As the TrypLE, it is possible to use, for example, TrypL Express (manufactured by Thermo Fisher Scientific, Inc. under the brand of Gibco), TrypLE Select (manufactured by Thermo Fisher Scientific, Inc. under the brand of Gibco), or the like.

The time during which a cell stripper is allowed to act on mesenchymal stem cells is preferably 10 minutes or more from the viewpoint of sufficiently detaching the cells. The time during which a cell stripper is allowed to act on mesenchymal stem cells is preferably 10 minutes to 120 minutes and more preferably a time within 10 minutes to 60 minutes. It may be 10 minutes to 50 minutes, 10 minutes to 40 minutes, 20 minutes to 60 minutes, 20 minutes to 50 minutes, or 20 minutes to 40 minutes.

The mesenchymal stem cell is a somatic stem cell derived from a mesodermal tissue (mesenchyme). The mesenchymal stem cells are known to be present in bone marrow, synovium, periosteum, adipose tissue, and muscle tissue, and they are known to have the ability to be differentiated into osteoblasts, cartilage cells, adipose cells, and muscle cells. In association with the differentiation of mesenchymal stem cells into cartilage cells, it is known that the addition of BMP or TGF-β to the culture solution promotes the differentiation of undifferentiated mesenchymal stem cells into cartilage cells, and the cartilage tissue can be regenerated under in vitro conditions.

The mesenchymal stem cell can be confirmed by detecting a molecule characteristic of the mesenchymal stem cell, for example, an enzyme, a receptor, a low-molecular-weight compound, or the like. Examples of the molecule characteristic of mesenchymal stem cells include, which are not limited to, CD73, CD90, CD105, and CD166, which are cell surface markers (positive markers). In addition, examples of the negative marker that are not expressed in mesenchymal stem cells include, but are not limited to, CD19, CD34, CD45, HLA-DR, CD11b, and CD14. It is noted that CD is an abbreviation for Clusters of d differentiation, and HLA-DR is an abbreviation for human leukocyte antigen-D-related. These positive markers and negative markers can be used to confirm that a cell is the mesenchymal stem cell. Although an immunological method can be used for the detection of these markers, the detection may be carried out by quantifying the mRNA amount of each molecule.

In the present specification, the synovium-derived mesenchymal stem cell is a stem cell contained in the synovium. The synovium-derived mesenchymal stem cell is a kind of mesenchymal stem cell. The synovium-derived mesenchymal stem cell can be detected, for example, by detecting CD90 positivity, CD45 negativity, or cartilage differentiation potency; however, the detection method is not particularly limited.

In a case where the synovium-derived mesenchymal stem cell produced by the above-described method is used as a therapeutic agent for arthrosis, this cell may be made into a pharmaceutical preparation in a form suitable for administration to an individual, by being mixed with a pharmaceutically acceptable carrier according to a conventional method. Examples of the carrier include physiological saline and distilled water for injection, which has been made to be isotonic by adding glucose and other auxiliary agents (for example, D-sorbitol, D-mannitol, sodium chloride, and the like). Further, a buffering agent (for example, a phosphate buffer solution or a sodium acetate buffer solution), a soothing agent (for example, benzalkonium chloride, procaine hydrochloride, or the like), a stabilizer (for example, human serum albumin, polyethylene glycol, or the like), a storage agent, an antioxidant, and the like may be blended.

The production method for a therapeutic agent for arthrosis according to the embodiment of the present invention may be further include preferably a step of sorting out synovium-derived mesenchymal stem cells having any one or more kinds of surface antigens of integrin β1 or platelet-derived growth factor receptor β.

Examples of the step of sorting out synovium-derived mesenchymal stem cells having any one or more kinds of surface antigens of integrin β1 or platelet-derived growth factor receptor β include managing an expression level of integrin β1 or platelet-derived growth factor receptor β.

The expression level of integrin β1 or platelet-derived growth factor receptor β refers to an expression level of a gene or protein of integrin β1 or platelet-derived growth factor receptor β. The expression level of integrin β1 or platelet-derived growth factor receptor β can be calculated as an absolute value or a relative value (a ratio, a difference, or the like with respect to a comparative control or a reference expression level).

The expression level of integrin β1 or platelet-derived growth factor receptor β can be measured by any method known to a person skilled in the art and carried out according to a conventional method. For the measurement of the expression level, an amount of mRNA which is a transcription product of a gene may be measured. The measurement of the mRNA amount is not particularly limited as long as it is a method that makes it possible to measure a desired amount of mRNA, where a known method can be appropriately selected and used among the known methods. For example, it is possible to use a gene amplification method in which an oligonucleotide that hybridizes to the gene encoding integrin β1 or platelet-derived growth factor receptor β is used as a primer or a hybridization method in which an oligonucleotide (or polynucleotide) that hybridizes to a gene encoding a specific protein molecule is used as a probe. Specific examples thereof include a reverse transcription polymerase chain reaction (RT-PCR) method, a real-time RT-PCR method, a DNA microarray method, a cell array method, a Northern blot method, a dot blot method, and an RNase protection assay.

The primer or probe that is used in the above-described measuring method is labeled, and the intensity of the signal of the label is investigated, whereby the amount of mRNA can be measured. The real-time RT-PCR method is preferable since RNA can be directly used as a sample and a gene can be quantified from the number of temperature cycles required for amplification, by optically measuring a gene amplification process. In addition, it is possible to standardize the expression level of the gene encoding integrin β1 or platelet-derived growth factor receptor β by using, as a control, the expression level of mRNA of glyceraldehyde-3-phosphate dehydrogenase (GAPDH), beta-actin, or the like, which is a housekeeping gene. It is noted that the primer and the probe, which are used in the above-described measuring methods, can be appropriately designed and prepared by those skilled in the art based on the information of the base sequence of the gene encoding the integrin β1 or the platelet-derived growth factor receptor β.

For the measurement of the expression level of integrin β1 or platelet-derived growth factor receptor β, it is possible to use, for example, a method of carrying out an immunological measurement by using an antibody or an antibody fragment against integrin β1 or platelet-derived growth factor receptor β. Specific examples thereof include flow cytometry, Western blotting, an enzyme-linked immuno-sorbent assay (ELISA), a radioimmunoassay (RIA), a fluorescent labeled antibody method, and a cell array method. Each of these measuring methods can also be carried out according to a protocol of a conventional method or a protocol obtained by appropriately modifying or changing a protocol of a conventional method.

For example, in a case where the expression level of integrin β1 or platelet-derived growth factor receptor β in cells is measured by flow cytometry, it is possible to sort out cells as synovium-derived mesenchymal stem cells having any one or more kinds of surface antigens of integrin β1 or platelet-derived growth factor receptor β in a case where the positive rate of integrin β1 or platelet-derived growth factor receptor β is preferably, 30% or more, 40% or more, 50% or more, 60% or more, 70% or more, 80% or more, or 90% or more.

In a case of sorting out synovium-derived mesenchymal stem cells having any one or more kinds of surface antigens of integrin β1 or platelet-derived growth factor receptor β, it is possible to sort out them by comparing, for example, the expression level of integrin β1 or platelet-derived growth factor receptor β in cells, which is measured by the above-described method, with a predetermined reference expression level. The reference expression level may be, for example, an expression level of the integrin β1 or platelet-derived growth factor receptor β in cells (a positive control) which already have been confirmed to have a constant quality or may be an expression level in cells (a negative control) which already have been confirmed not to have a constant quality.

The expression level of integrin β1 or platelet-derived growth factor receptor β is compared with the reference expression level to sort out cells in which the expression level of integrin β1 or platelet-derived growth factor receptor β in cells is equal to or higher than the expression level of the positive control, whereby the cells can be used as a therapeutic agent for arthrosis.

In addition, a cut off value of the expression level of integrin β1 or platelet-derived growth factor receptor β may be set in advance, and the expression level of integrin β1 or platelet-derived growth factor receptor β measured in cells may be compared with the cut off value. The cut off value can be set as, for example, an expression level of integrin β1 or platelet-derived growth factor receptor β, and an expression level of integrin β1 or platelet-derived growth factor receptor β, at which a desired therapeutic effect is provided based on a regression line showing a correlation with the therapeutic effect. For example, cells in which the expression level of integrin β1 or platelet-derived growth factor receptor β in cells is equal to or higher than the cut off value are sorted out, whereby the cells can be used as a therapeutic agent for arthrosis.

The therapeutic agent for arthrosis according to the embodiment of the present invention can be used for a joint medical treatment. Examples of the joint medical treatment include a medical treatment for a disease associated with injury, injury, or inflammation of the joint, and examples of the medical treatment include a medical treatment for a joint disease resulting from degeneration and/or inflammation of the connective tissue such as cartilage, or a non-inflammatory joint disease. Examples of the joint medical treatment include a medical treatment for a disease selected from the group consisting of meniscus injury, traumatic cartilage injury, osteochondritis dissecans, aseptic osteonecrosis, osteoarthritis (for example, knee osteoarthritis), rheumatoid arthritis (for example, chronic rheumatoid arthritis), gout, reactive arthritis, psoriatic arthritis, juvenile arthritis, inflammatory arthritis, and articular cartilage defect, which are not limited to these diseases.

The medical treatment method for a joint, which uses the therapeutic agent for arthrosis according to the embodiment of the present invention, includes:
a step of transplanting the therapeutic agent for arthrosis according to the embodiment of the present invention so that a cartilage injury part or a meniscus injury part is covered by synovium-derived mesenchymal stem cells, and
a step of differentiating the synovium-derived mesenchymal stem cells contained in the therapeutic agent for arthrosis into a cartilage cell to regenerate cartilage tissue in situ at a cartilage injury part or a meniscus injury part.

In a case of transplanting the therapeutic agent for arthrosis according to the embodiment of the present invention to a patient, it is preferable to apply, per cartilage injury part or meniscus injury part, synovium-derived mesenchymal stem cells of 2.0 × 10⁷ to 1.0 × 10¹¹ cells, 2.5 × 10⁷ to 1.0 × 10¹¹ cells, 3.0 × 10⁷ to 1.0 × 10¹¹ cells, 4.0 × 10⁷ to 1.0 × 10¹¹ cells, 2.5 × 10⁷ to 1.0 × 10¹⁰ cells, 2.5 × 10⁷ to 1.0 × 10⁹ cells, or 2.5 × 10⁷ to 1.0 × 10^{g} cells, or synovium-derived mesenchymal stem cells of 2.0 × 10⁷ to 1.0 × 10⁸ cells, in order to efficiently treat medically the cartilage injury part or the meniscus injury part.

By transplanting the synovium-derived mesenchymal stem cells into the cartilage injury part or the meniscus injury part, the cartilage injury part or the meniscus injury part is covered with the synovium-derived mesenchymal stem cells. The transplantation of the /synovium-derived mesenchymal stem cell can be carried out by open surgery or by arthroscopic surgery. In order to minimize the invasion as much as possible, it is preferable to arthroscopically transplant the synovium-derived mesenchymal stem cell.

The cartilage injury part or the meniscus injury part may be covered with a suspension of synovium-derived mesenchymal stem cells or may be covered with a cell sheet of synovium-derived mesenchymal stem cells. For example, a gel having bioabsorbability such as gelatin or collagen can be used as a gel-like substance. The synovium-derived mesenchymal stem cells have a high ability to adhere to the cartilage injury part or the meniscus injury part.

In a case of a medical treatment for cartilage injury, the minimally invasive method according to the embodiment of the present invention is characterized in that the cartilage injury part is covered with synovium-derived mesenchymal stem cells, and it includes the following steps:
holding the body position so that the cartilage injury part faces upward;
placing a cell sheet of synovium-derived mesenchymal stem cells, a suspension of synovium-derived mesenchymal stem cells, or a gel-like substance containing synovium-derived mesenchymal stem cells on the surface of the cartilage injury part; and
holding the body position for a specific period of time to allow the synovium-derived mesenchymal stem cells to adhere to the surface of the cartilage injury part.

In a case of a medical treatment for meniscus injury, the minimally invasive method according to the embodiment of the present invention is characterized in that the meniscus injury part is covered with synovium-derived mesenchymal stem cells, and it includes the following steps:
holding the body position so that the meniscus injury part faces downward;
injecting the suspension of synovium-derived mesenchymal stem cells into a knee joint; and
holding the body position for a specific period of time to allow the synovium-derived mesenchymal stem cells to adhere to the meniscus injury part.

In order to reliably allow the synovium-derived mesenchymal stem cells to adhere to the surface of the cartilage injury part or meniscus injury part, the transplanted synovium-derived mesenchymal stem cells are held on the surface of the cartilage injury part or meniscus injury part for at least 10 minutes and preferably 15 minutes. In order to achieve this, the body position is held for at least 10 minutes and preferably 15 minutes in order to achieve that the cartilage injury part or the meniscus injury part faces upward and that the synovium-derived mesenchymal stem cells are held in the cartilage injury part or the meniscus injury part, which faces upward.

In order to further strengthen the adhesion of the synovium-derived mesenchymal stem cells to the cartilage injury part or the meniscus injury part, the cartilage injury part or the meniscus injury part associated with the synovium-derived mesenchymal stem cells can be covered with the periosteum. The operation is completed after holding the synovium-derived mesenchymal stem cells on the surface of the cartilage injury part or the surface of the meniscus injury part for at least 10 minutes.

In the present invention, the transplanted synovium-derived mesenchymal stem cells are differentiated into cartilage cells at the cartilage injury part or the meniscus injury part and the cartilage tissue is regenerated in situ at the cartilage injury part or the meniscus injury part.

During the in situ cartilage formation process of the synovium-derived mesenchymal stem cells, the cartilage tissue is regenerated depending on the local microenvironment (the nutrient supply, the cytokine environment, and the like), and thus no external operation is required. As a result of the in situ cartilage formation of synovium-derived mesenchymal stem cells, the cartilage tissue is regenerated at the cartilage injury part or the meniscus injury part to repair the injury. Then, in a case of cartilage injury, a bone region, a boundary between the cartilage and the bone, a central part of the cartilage, a surface region, and a region adjacent to the original cartilage are formed as the original cartilage tissue, or in a case of the meniscus injury, meniscus cartilage is formed.

The present invention will be further specifically described with reference to Examples; however, the present invention is not limited by Examples.

### Examples

### <Example 1> Preparation of rat synovium-derived mesenchymal stem cell

A LEW/CrlCrlj rat was used to establish rat synovium-derived mesenchymal stem cells. To a culture medium of αMEM no nucleosides (Gibco Cat. No. 12561056), collagenase V (Sigma Cat. No. C9263) was added so that the concentration thereof was 2 or 3 mg/mL, and the synovial tissue collected under isoflurane anesthesia was reacted in the culture medium at 37°C for 2 hours. A cooled culture medium was added to stop the reaction, and the residual tissue was removed by being passed through a 40 µm cell strainer. The recovered cells were seeded in a cell culture flask and cultured at a CO₂ concentration of 5% and 37°C in αMEM no nucleosides to which Fetal Bovine Serum (Gibco Cat. No. 10270106) was added to a final concentration of 20%, L-glutamine 200 mmol/L (Gibco Cat. # 25030081) was added to a final concentration of 1%, and Antibiotic-Antimycotic (100X) (Gibco Cat. No. 15240062) was added to a final concentration of 1%. After culturing for 8 days, the culture medium in the flask was discarded, and washing was carried out twice with phosphate buffered saline (PBS). Then, TrypLE Express (Gibco Cat. No. 12604-013) was added thereto, and the cells were allowed to stand in an incubator of 37°C for 5 minutes and recovered as synovium-derived mesenchymal stem cells. The supernatant was discarded by centrifugation and substituted with COS-banker (COSMO BIO Cat. No. COS-CFM01) to prepare a stock of the frozen rat synovium-derived mesenchymal stem cells.

### <Example 2> Suppression of extracellular matrix adhesion ability of rat synovium-derived mesenchymal stem cell by inhibition of integrin β1

For the preparation of rat synovium-derived mesenchymal stem cells in which integrin β1 was inhibited, a frozen stock of the rat synovium-derived mesenchymal stem cells prepared in Example 1 was woken up and cultured at a CO₂ concentration of 5% and 37°C for one week using αMEM no nucleosides to which Fetal Bovine Serum was added to a final concentration of 20%, L-glutamine 200 mmol/L was added to a final concentration of 1%, and Antibiotic-Antimycotic (100X) was added to a final concentration of 1%, and the recovered cells were suspended in a reaction solvent of PBS containing 2% FBS. 12 µg of Purified anti-mouse/rat CD29 Antibody (BioLegend Cat. No. 102202) per number of cells of 5 × 10⁶ cells was added thereto and reacted with cells for 1 hour under ice-cooling, and then the cells were recovered (integrin β1-rSMSC). As a control treatment in which the inhibition of integrin β1 was not carried out, Purified Armenian Hamster IgG Isotype Ctrl (BioLegend Cat. No. 400902) and reacted with cells for 1 hour under ice-cooling, and then the cells were recovered (IgG-rSMSC). In addition, untreated cells (Non-treated-rSMSC) that had undergone the same reaction only with the reaction solvent were provided and subjected to the following adhesion treatment.

In order to confirm that the integrin β1 was inhibited, the function of adhering to an extracellular matrix, which is one of the functions of the integrin β1, was checked. Washing was carried out with PBS containing 10 mmol/L MgCl₂·6H₂O (hereinafter, PBS (+)) as an extracellular matrix adhesion reaction, and cells were suspended again in PBS (+), adjusted to 1 × 10⁵ cells/10 µL/well, and seeded on a collagen type I Cellware 8-well culture slide (Corning Cat. No. 354630). After being allowed to stand at room temperature for 10 minutes, the cells were washed with PBS (+). Thereafter, an observation was carried out with a microscope (OLIMPUS Cat. No. IX71) using an objective lens at a magnification of 10 times. In addition, an image of one visual field at a place where adhered cells were most frequently observed was acquired, and the number of adhered cells was measured.

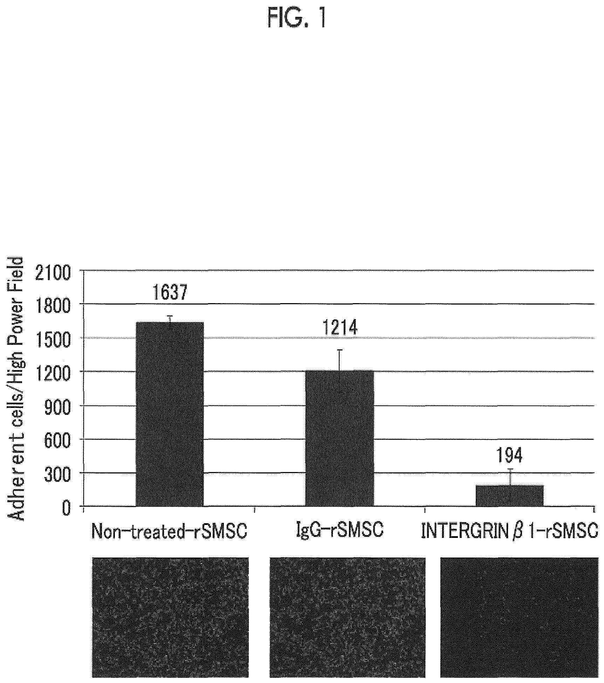

The results are shown in Fig. 1. The number of adhered cells was 1,637 cells for the Non-treated-rSMSC, 1,214 cells for the IgG-rSMSC, and 194 cells for the integrin β1-rSMSC, and a significant decrease in the number of adhered cells was observed due to the inhibition of integrin β1 of the cells. From this result, it was confirmed that integrin β1 in the rat synovial stem cells can be inhibited by the treatment with Purified anti-mouse/rat CD29 Antibody.

### <Example 3> Suppression of cell proliferation ability of rat synovium-derived mesenchymal stem cell by inhibition of PDGFRb

For the preparation of rat synovium-derived mesenchymal stem cells in which PDGFRb was inhibited, the frozen stock prepared in Example 1 was woken up and cultured at a CO₂ concentration of 5% and 37°C for one week using αMEM no nucleosides to which Fetal Bovine Serum was added to a final concentration of 20%, L-glutamine 200 mmol/L was added to a final concentration of 1%, and Antibiotic-Antimycotic (100X) was added to a final concentration of 1%, and the recovered cells were suspended in a reaction solvent of PBS containing 2% FBS.

40 or 120 µg of Anti-PDGF Receptor β Human Goat-Poly (R & D Systems Cat. No. AF385) per number of cells of 1 × 10⁶ cells was reacted with cells for 1 hour under ice-cooling. Then, the cells were seeded on a 96-well plate (Corning Cat No. 353072) at 1,000 cells/well, and the culture was started at a CO₂ concentration of 5% and 37°C (PDGFRb-rSMSC). As a control treatment in which the inhibition of PDGFRb was not carried out, Normal Goat IgG Control (R & D Systems Cat. No. AB-108-C) was reacted with cells for 1 hour under ice-cooling, and the cells were seeded on a 96-well plate at 1,000 cells/well (IgG-rSMSC). In addition, Non-treated-rSMSC, where cells were seeded at 1,000 cells/well in a 96-well plate without carrying out a reaction, was also provided.

In order to confirm that PDGFRb was inhibited, the cell proliferation ability, which is a function of PDGFRb, was checked. Cells were cultured at a CO₂ concentration of 5% and 37°C, and the cell proliferation properties were quantitatively evaluated by an ATP assay using Cell Titer Glo (Promega Cat. No. G7571) on the 6th day of culture.

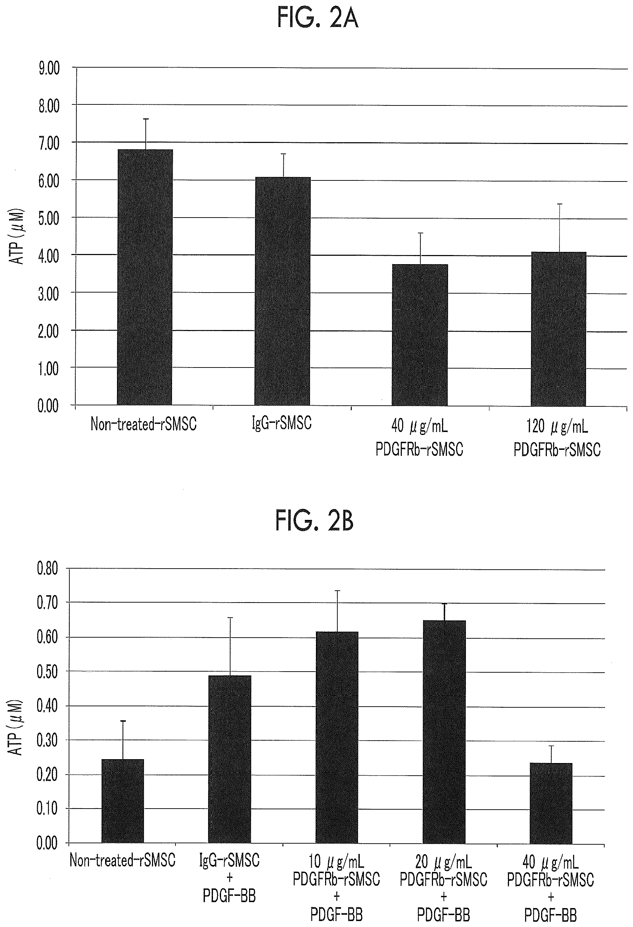

The results are shown in Fig. 2a. The ATP concentration of the 40 µg/mL PDGFRb-rSMSC was 3.78 ± 0.84 µmol/L, the ATP concentration of the 120 µg/mL PDGFRb-rSMSC was 4.12 ± 1.29 µmol/L, the ATP concentration of the IgG-rSMSC was 6.08 ± 0.63 µmol/L, the ATP concentration of the Non-treated-rSMSC was 6.81 ± 0.82 µmol/L, and a significant suppression of cell proliferation was observed due to the inhibition of PDGFRb. From this result, it was confirmed that the proliferation of the rat synovial stem cells can be suppressed by the treatment with Anti-PDGF Receptor β Human Goat-Poly.

In order to check the ligand specificity of PDGFRb, the frozen stock prepared in Example 1 was woken up for the preparation of rat synovium-derived mesenchymal stem cells in which PDGFRb was inhibited, and cultured at a CO₂ concentration of 5% and 37°C for one week using αMEM no nucleosides to which Fetal Bovine Serum was added to a final concentration of 20%, L-glutamine 200 mmol/L was added to a final concentration of 1%, and Antibiotic-Antimycotic (100X) was added to a final concentration of 1%, and the recovered cells were suspended in a reaction solvent of PBS containing 2% FBS.

10, 20, or 40 µg of Anti-PDGF Receptor β Human Goat-Poly (R & D Systems Cat. No. AF385) per number of cells of 1 × 10⁶ cells was reacted with cells for 1 hour under ice-cooling. Then, the cells were seeded on a 96-well plate (Corning Cat No. 353072) at 1,000 cells/well, and the culture was started at a CO₂ concentration of 5% and 37°C (PDGFRb-rSMSC). As a control treatment in which the inhibition of PDGFRb was not carried out, Normal Goat IgG Control (R & D Systems Cat. No. AB-108-C) was reacted with cells for 1 hour under ice-cooling, and the cells were seeded on a 96-well plate at 1,000 cells/well (IgG-rSMSC). In addition, Non-treated-rSMSC, where cells were seeded at 1,000 cells/well in a 96-well plate without carrying out a reaction, was also provided.

On the day after cell seeding, the culture supernatant was discarded, the culture medium was exchanged to αMEM no nucleosides (Gibco Cat. No. 10270106) to which Fetal Bovine Serum was added to a final concentration of 0.5%, L-glutamine 200 mmol/L was added to a final concentration of 1%, Antibiotic-Antimycotic (100X) was added to a final concentration of 1%, PDGF-BB and Rat, Recombinant (R & D systems Cat. No. 520-BB-050) were added to a concentration of 4 ng/mL. As the experimental level, Anti-PDGF Receptor β Human Goat-Poly (R & D Systems Cat. No. AF385) was added to a final concentration of 10, 20, or 40 µg/mL, and the total amount of the culture medium was set to 100 µL. Normal Goat IgG Control (R & D Systems Cat. No. AB-108-C) was added to the positive control, and the total amount of the culture medium was set to 100 µL. No antibody was added to the negative control, and only 100 µL of the culture medium was added thereto. On the 6th day of culture, the cell proliferation properties were quantitatively evaluated by an ATP assay using Cell Titer Glo (Promega Cat. No. G7571).

The results are shown in Fig. 2b. The ATP concentration of the 10 µg/mL PDGFRb-rSMSC was 0.62 ± 0.12 µmol/L, the ATP concentration of the 20 µg/mL PDGFRb-rSMSC was 0.65 ± 0.05 µmol/L, the ATP concentration of the 40 µg/mL PDGFRb-rSMSC was 0.24 ± 0.05 µmol/L, the ATP concentration of the IgG-rSMSC was 0.49 ± 0.17 µmol/L, the ATP concentration of the Non-treated-rSMSC was 0.24 ± 0.11 µmol/L, and the 40 µg/mL PDGFRb-rSMSC showed a significant decrease in cell proliferation with respect to the IgG-rSMSC. From this, it was confirmed that PDGFRb in the rat synovial stem cells can be inhibited in a ligand-specific manner by the treatment with Anti-PDGF Receptor β Human Goat-Poly.

### <Example 4> Preparation of Col2A1-deleted rat synovium-derived mesenchymal stem cell

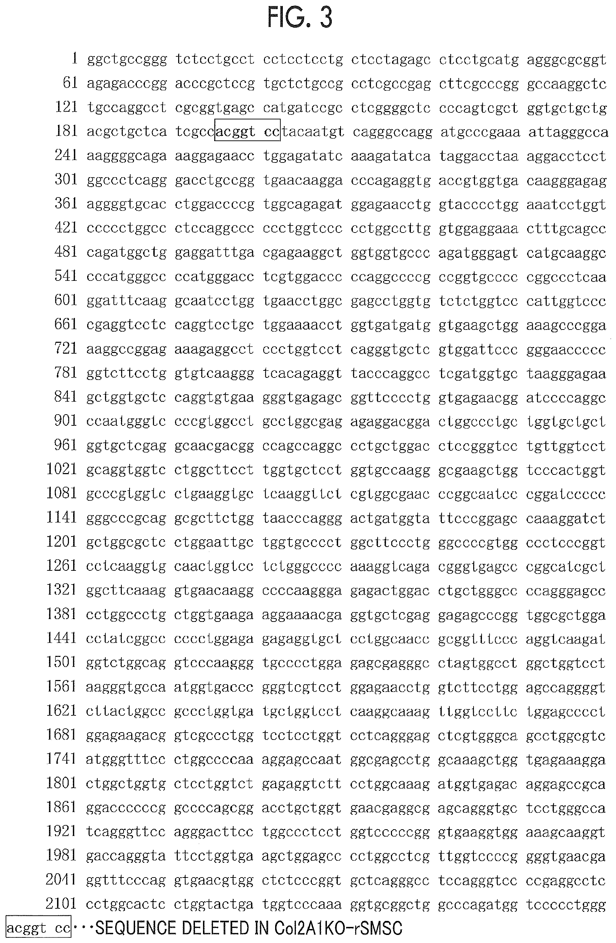

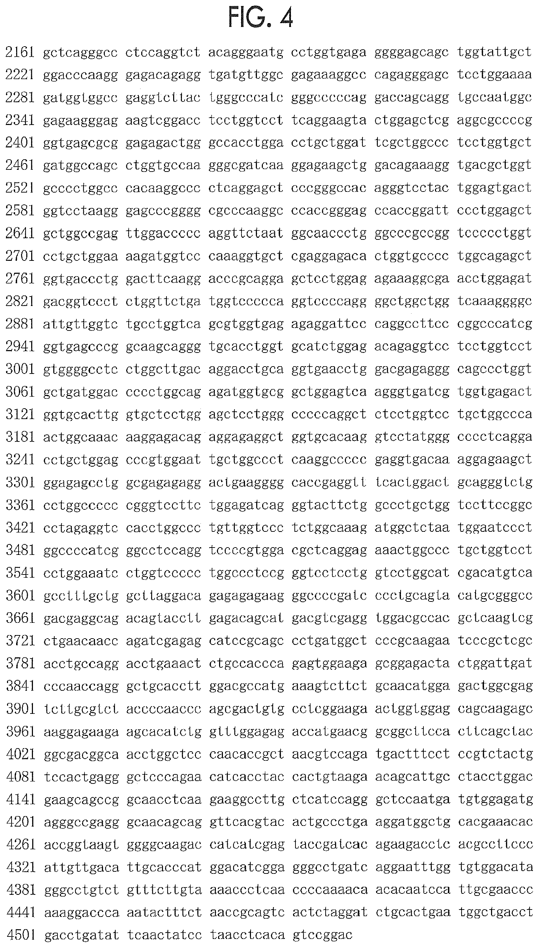

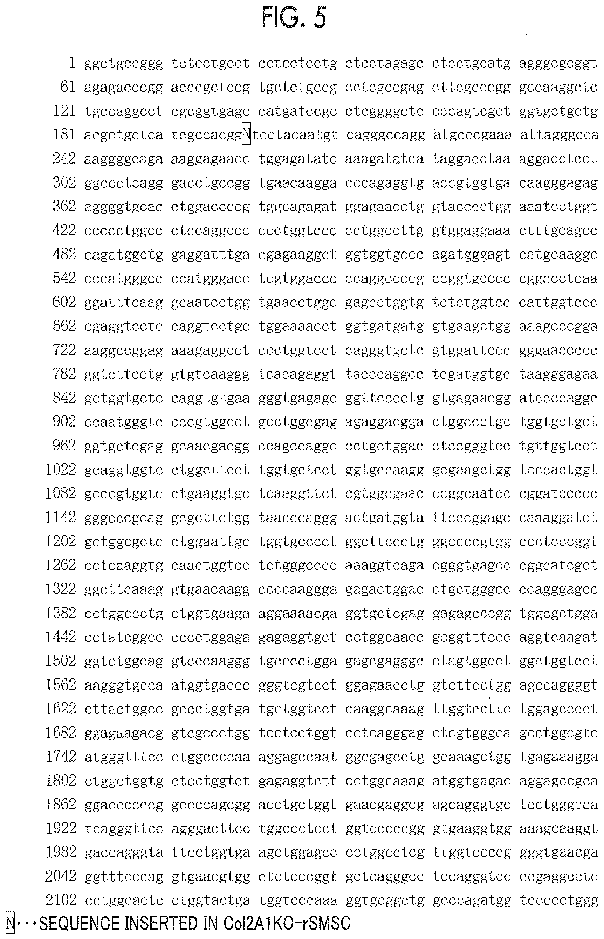

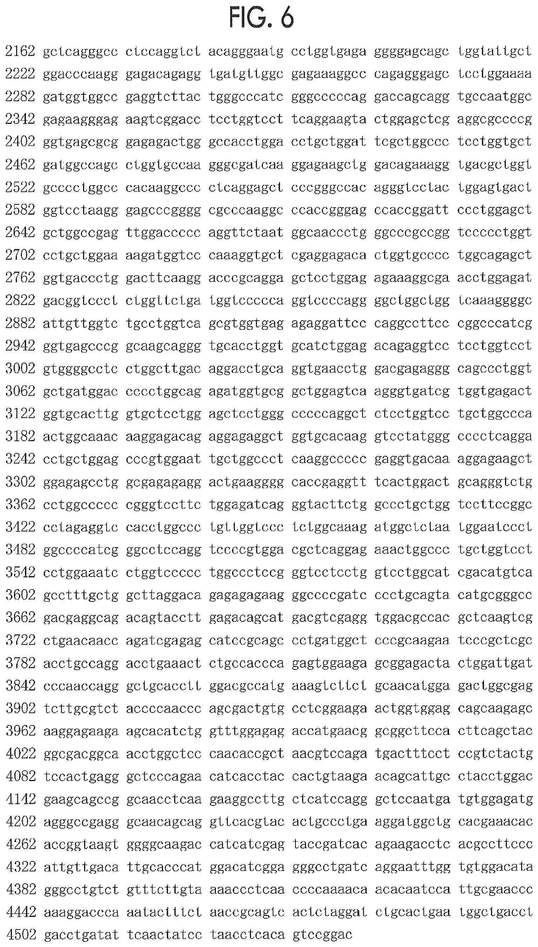

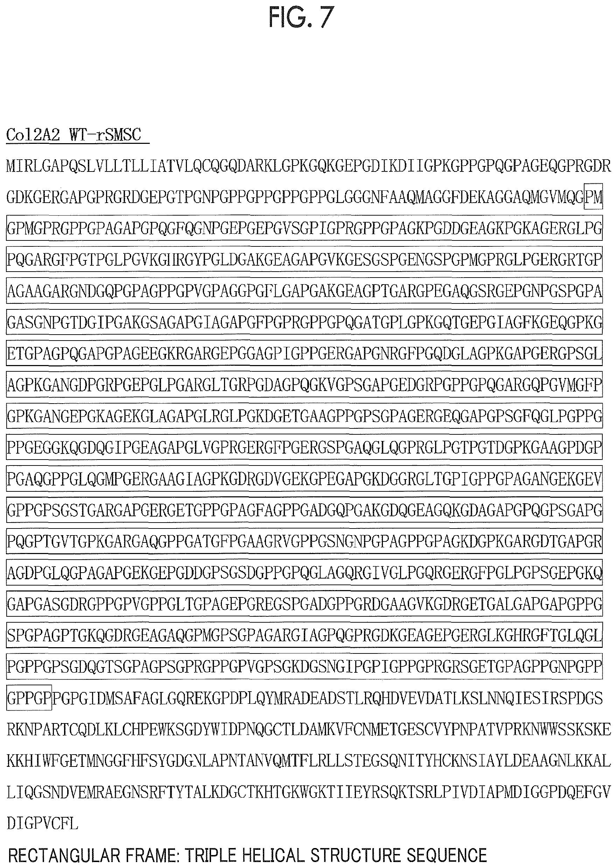

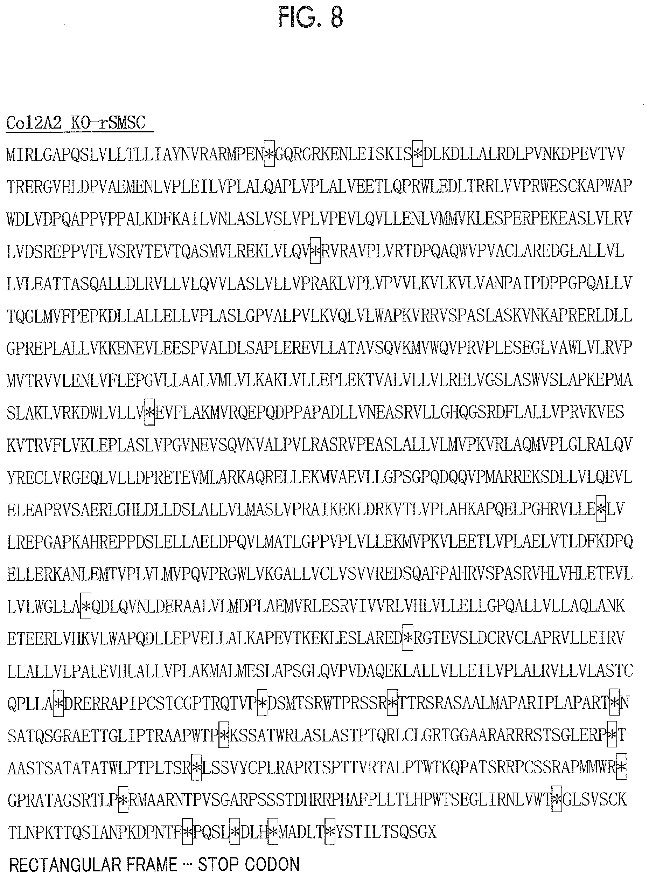

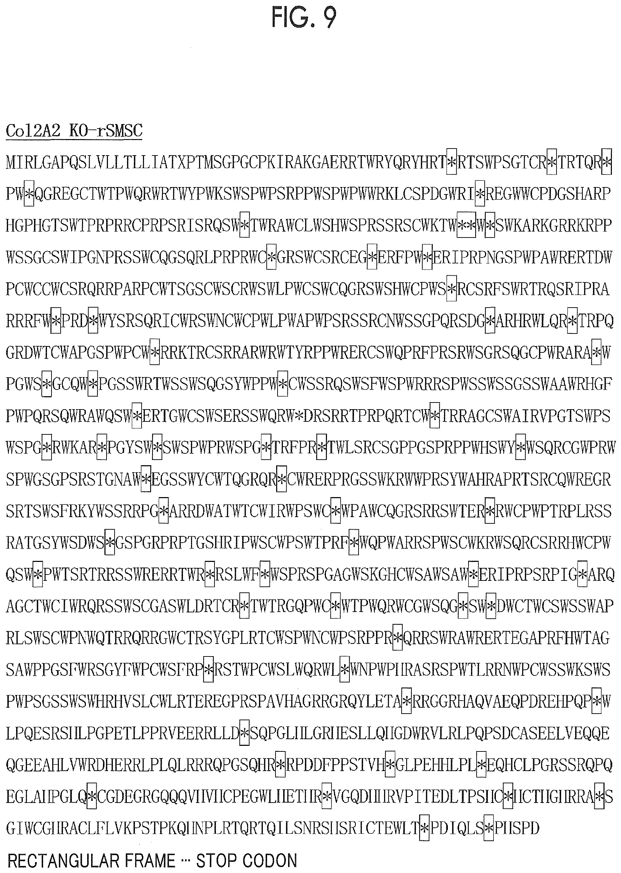

The rat synovium-derived mesenchymal stem cells prepared in Example 1 were subjected to an operation of deletion of a gene of Col2A1, and the deletion of the Col2A1 gene was checked according to the Sanger sequence analysis. Fig. 3, Fig. 4, Fig. 5, and Fig. 6 each show the base sequence of Col2A1 for the wild type (Col2A1WT-rSMSC) and the deletion type (Col2A1KO-rSMSC) of the Col2A1 gene of the rat synovial stem cell, and Fig. 7, Fig. 8, and Fig. 9 each show an amino acid sequence that is translated based on this sequence. The base sequence of Col2A1 for the wild type (Col2A1WT-rSMSC) of the Col2A1 gene of the rat synovial stem cell is set forth in SEQ ID NO: 1, the base sequence of Col2A1 for the deletion type (Col2A1KO-rSMSC) of one allele of the Col2A1 gene on the chromosome of the rat synovial stem cell is set forth in SEQ ID NO: 2, the base sequence of Col2A1 for the deletion type (Col2A1KO-rSMSC) of the other allele of the Col2A1 gene on the chromosome of the rat synovial stem cell is set forth in SEQ ID NO: 3. The amino acid sequence of Col2A1 for the wild type (Col2A1WT-rSMSC) is set forth in SEQ ID NO: 4, and The amino acid sequence of Col2A1 for the deletion type (Col2A1KO-rSMSC) is set forth in SEQ ID NO: 5 and SEQ ID NO: 6. As a result, it was found that Col2A1KO-rSMSC is a hetero frame shift mutant that has a DNA having a deletion of bases from the 55th position to the 62nd position and a DNA having an insertion of a base at the 59th position, where the position is counted from ATG, which is an amino acid translation initiation codon, as a starting point. It was found that the base sequence is such a base sequence from which a mutant having 28 amino acid residues is translated since in one of the alleles, the mutation occurs in the sequence from the amino acid at the 19th position due to the base deletion, and a stop codon is introduced at the 29th position, although the number of amino acids should be originally 1,419 amino acids. It was found that the base sequence is such a base sequence from which a mutant having 49 amino acid residues is translated since in the other of the alleles, the mutation occurs in the sequence from the amino acid at the 20th position due to the base deletion, and a stop codon is introduced at the 50th position, although the number of amino acids should be originally 1,419 amino acids. Due to the fact that in the base sequence that gives this mutant, a sequence from the 133rd amino acid to the 1,146th amino acid, which is a triple helical structure domain that is an important functional domain of Col2A1, is not translated, it was determined that the synovial stem cell (Col2A1KO-rSMSC) having a gene sequence in which the function of Col2A1 is deleted has been acquired.

### <Comparative Example 1> Preparation of CD120a-deleted rat synovium-derived mesenchymal stem cell

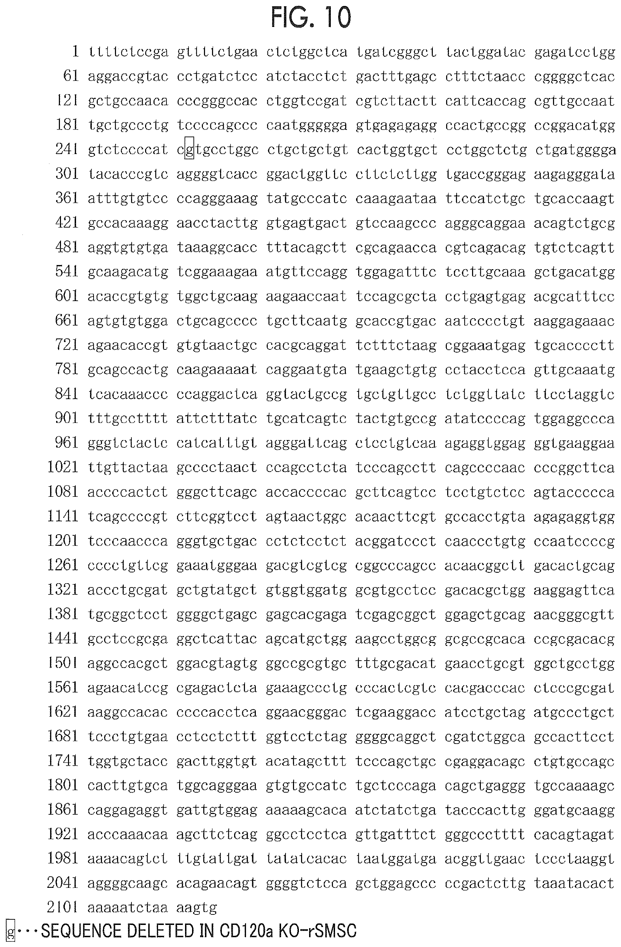

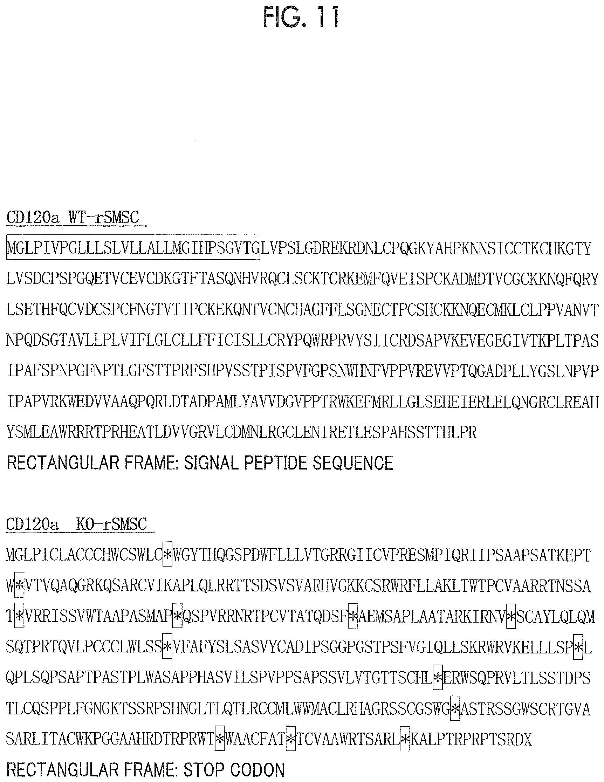

The rat synovium-derived mesenchymal stem cells prepared in Example 1 were subjected to an operation of deletion of a gene of CD120a, and the deletion of the CD120a gene was checked according to the Sanger sequence analysis. Fig. 10 and Fig. 11 respectively show the base sequence of CD120a for the wild type (CD120aWT-rSMSC) and the deletion type (CD120aKO-rSMSC) of the CD120a gene of the rat synovial stem cell, and an amino acid sequence that is translated based on the base sequence. The base sequence of CD120a for the wild type (CD120aWT-rSMSC) and the deletion type (CD120aKO-rSMSC) of the CD120a gene of the rat synovial stem cell is set forth in SEQ ID NO: 7, the amino acid sequence of the wild type of the CD120a gene (CD120aWT-rSMSC) is set forth in SEQ ID NO: 8, and the amino acid sequence of the deletion type of the CD120a gene (CD120aKO-rSMSC) is set forth in SEQ ID NO: 9. As a result, it was found that CD120aKO-rSMSC is a frame shift mutant that has a DNA having a deletion of a base at the 16th position, where the position is counted from ATG, which is an amino acid translation initiation codon, as a starting point. It was found that the base sequence is such a base sequence from which a mutant having 19 amino acid residues is translated since the mutation occurs in the sequence from the amino acid at the 6th position due to the base deletion, and a stop codon is introduced at the 19th position, although the number of amino acids should be originally 461 amino acids. Due to the fact that in the base sequence that gives the mutant, a sequence of the protein region constituting CD120a is not translated, it was determined that the synovial stem cell (CD120aKO-rSMSC) having a gene sequence in which the function of CD120a is deleted has been acquired.

### <Comparative Example 2> Preparation of CD106-deleted rat synovium-derived mesenchymal stem cell

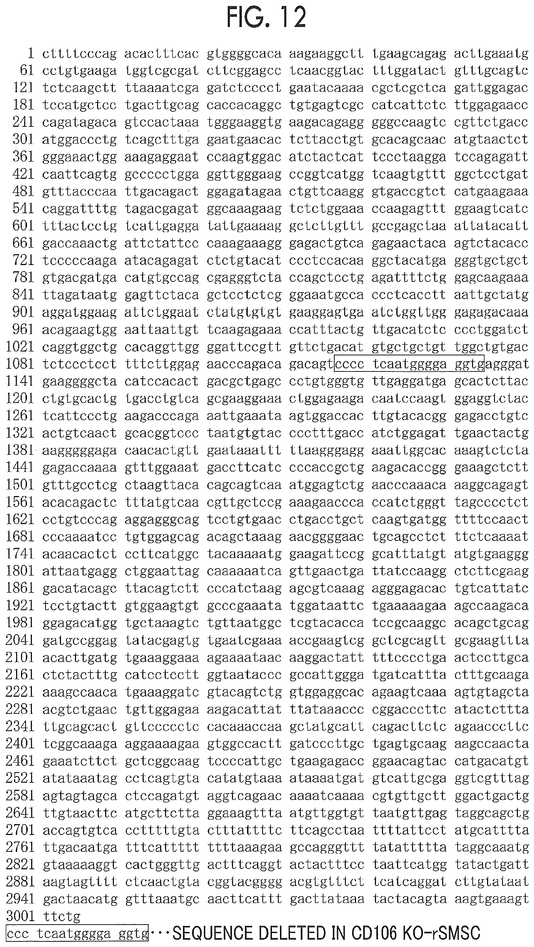

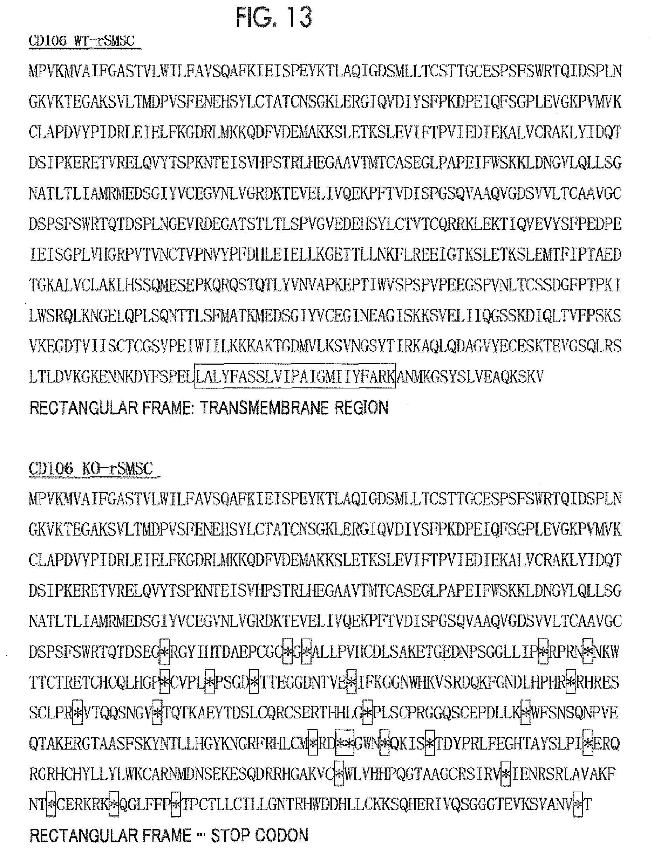

The rat synovium-derived mesenchymal stem cells prepared in Example 1 were subjected to an operation of deletion of a gene of CD106, and the deletion of the CD106 gene was checked according to the Sanger sequence analysis. Fig. 12 and Fig. 13 respectively show the base sequence of CD106 for the wild type (CD106WT-rSMSC) and the deletion type (CD106KO-rSMSC) of the CD106 gene of the rat synovial stem cell, and an amino acid sequence that is translated based on the base sequence. The base sequence of CD106 for the wild type (CD106WT-rSMSC) and the deletion type (CD106KO-rSMSC) of the CD106 gene of the rat synovial stem cell is set forth in SEQ ID NO: 10, the amino acid sequence of the wild type of the CD106 gene (CD106WT-rSMSC) is set forth in SEQ ID NO: 11, and the amino acid sequence of the deletion type of the CD106 gene (CD106KO-rSMSC) is set forth in SEQ ID NO: 12. As a result, it was found that CD106KO-rSMSC is a frame shift mutant that has a DNA having a deletion of bases from the 1,059th position to the 1,076th position, where the position is counted from ATG, which is an amino acid translation initiation codon, as a starting point. It was found that the base sequence is such a base sequence from which a mutant having 355 amino acid residues is translated since the mutation occurs in the sequence from the amino acid at the 354th position due to the base deletion, and a stop codon is introduced at the 356th position, although the number of amino acids should be originally 739 amino acids. Due to the fact that in the base sequence that gives the mutant, a sequence from the 699th amino acid to the 720th amino acid, which is the transmembrane region of CD106, is not translated, it was determined that the synovial stem cell (CD106KO-rSMSC) having a gene sequence in which the function of CD106 is deleted has been acquired.

### <Example 5> Suppression of cartilage differentiation potency of rat synovium-derived mesenchymal stem cells from which Col2A1 has been deleted

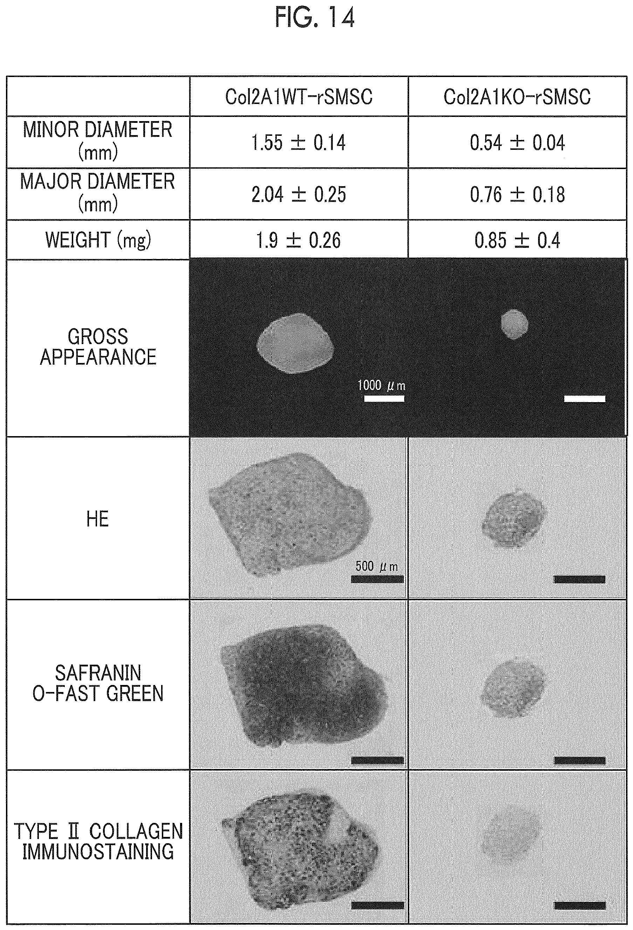

Col2A1 is one of the cartilage constitutional components. In order to check the deletion of Col2A1 at the level of cell function, the cartilage differentiation potency of Col2A1KO-rSMSC was investigated. 2.5 × 10⁵ cells of the Col2A1KO-rSMSC prepared in Example 4 were suspended in DMEM high glucose (Thermo Cat. No. 11965092) to which TGF-β3 (R & D Systems Cat. No. 243-B3-002) was added to a final concentration of 10 ng/mL, Dexamethasone (Wako Cat. No. 041-18861) was added to a final concentration of 3.92 µg/mL, L-Ascorbic Acid 2-phosphate (Cayman Chemical Cat. No. 16457) was added to a final concentration of 50 µg/mL, L-Proline (MP Biomedicals Cat. No. 194728) was added to a final concentration of 40 µg/mL, Sodium Pyruvate (Invitrogen Cat. No. 11360070) was added to a final concentration of 1 µg/mL, ITS-X supplement (x100) (Wako Cat. No. 094-06761) was added to a final concentration of 1%, and BMP-2 (R & D Systems Cat. No. 355-BM-010) was added to a final concentration of 0.5 µg/mL, centrifugation was carried out at 450 g for 10 minutes, and then the culture was started at a CO₂ concentration of 5% and 37°C (PDGFRb-rSMSC) to induce cartilage differentiation. In addition, as a control cell, the Col2A1WT-rSMSC was subjected to cartilage differentiation induction in the same manner. After culturing for 3 weeks, the diameter and weight of the cell aggregates were measured, and the cartilage differentiation potency was evaluated from the tissue staining of the cell aggregates. The results are shown in Fig. 14. In the Col2A1WT-rSMSC, the minor axis was 1.55 ± 0.14 mm, the major axis was 2.04 ± 0.25 mm, and the weight was 1.9 ± 0.26 mg, whereas in the Col2A1KO-rSMSC, the minor axis was 0.54 ± 0.04 mm, the major axis was 0.76 ± 0.18 mm, and the weight was 0.85 ± 0.4 mg, and thus there was a significant decrease in the size and weight of the cartilage. Due to the fact that Col2A1 is a cartilage constitutional component, it can be seen that the deletion of Col2A1 has reduced the size and weight of the cartilage. In addition, in the Col2A1KO-rSMSC, it was found that the stainability has disappeared in the Safranin O-Fast green staining and the type II collagen immunostaining. That is, it was shown that the deletion of Col2A1 affects not only the disappearance of the production ability of type II collagen but also the production of a cartilage substrate of mucopolysaccharides. This suggests that the type II collagen contributes not only to the formation of the cartilage tissue skeleton but also to the action of induction of cartilage differentiation/substrate production.

### <Comparative Example 3> Suppression of cartilage differentiation potency of rat synovium-derived mesenchymal stem cells from which CD120a has been deleted

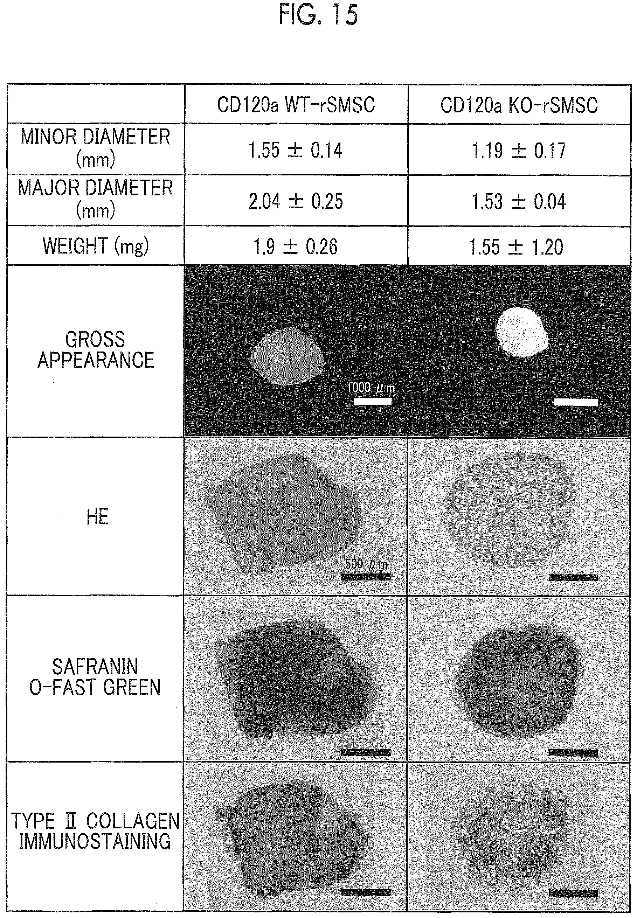

The cartilage differentiation potency of the CD120aKO-rSMSC prepared in Comparative Example 1 was investigated. The cartilage differentiation induction was carried out in the same differentiation culture medium and culture conditions as those in Example 5. In addition, as a control cell, the CD120aWT-rSMSC was subjected to cartilage differentiation induction in the same manner. After culturing for 3 weeks, the diameter and weight of the cell aggregates were measured, and the cartilage differentiation potency was evaluated from the tissue staining of the cell aggregates. The results are shown in Fig. 15. In the CD120aWT-rSMSC, the minor axis was 1.55 ± 0.14 mm, the major axis was 2.04 ± 0.25 mm, and the weight was 1.9 ± 0.26 mg, whereas in the CD120aKO-rSMSC, the minor axis was 1.19 ± 0.17 mm, the major axis was 1.53 ± 0.04 mm, and the weight was 1.55 ± 1.20 mg, and thus there was no significant decrease in the size and weight of the cartilage. In addition, due to the fact that the difference in staining properties due to the deletion of CD120a was not observed in the Safranin O-Fast green staining and the type II collagen immunostaining, CD120a is conceived to be a molecule that does not contribute to the cartilage differentiation potency of cells.

### <Comparative Example 4> Suppression of cartilage differentiation potency of rat synovium-derived mesenchymal stem cells from which CD106 has been deleted

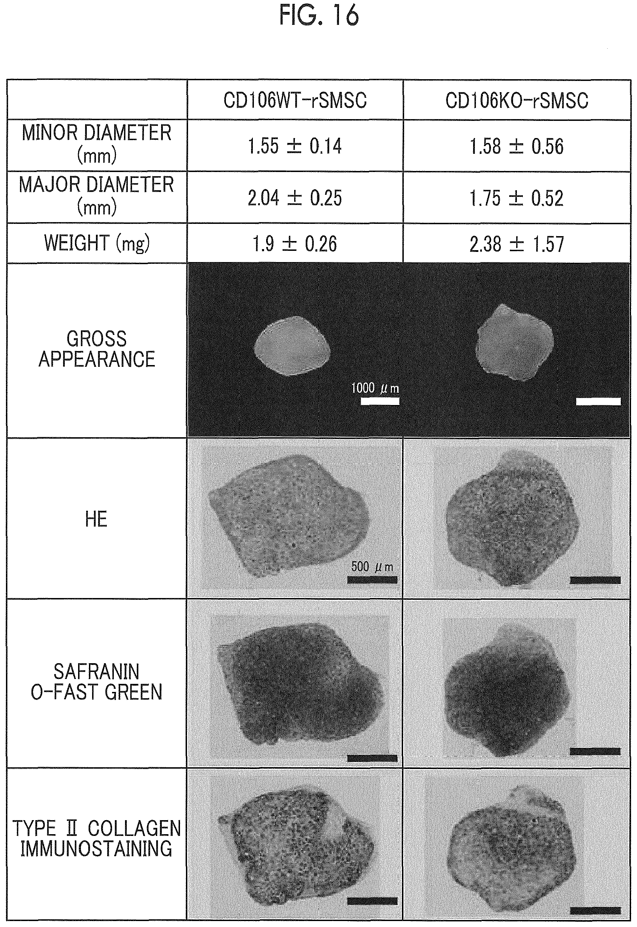

The cartilage differentiation potency of the CD106KO-rSMSC prepared in Comparative Example 2 was investigated. The cartilage differentiation induction of 2.5 × 10⁵ cells of the CD106KO-rSMSC was carried out in the same differentiation culture medium and culture conditions as those in Example 5. In addition, as a control cell, the CD106WT-rSMSC was subjected to cartilage differentiation induction in the same manner. After culturing for 3 weeks, the diameter and weight of the cell aggregates were measured, and the cartilage differentiation potency was evaluated from the tissue staining of the cell aggregates. The results are shown in Fig. 16. In the CD106WT-rSMSC, the minor axis was 1.55 ± 0.14 mm, the major axis was 2.04 ± 0.25 mm, and the weight was 1.9 ± 0.26 mg, whereas in the CD106KO-rSMSC, the minor axis was 1.58 ± 0.56 mm, the major axis was 1.75 ± 0.52 mm, and the weight was 2.38 ± 1.54 mg, and thus there was no significant decrease in the size and weight of the cartilage. In addition, due to the fact that the difference in staining properties due to the deletion of CD106 was not observed in the Safranin O-Fast green staining and the type II collagen immunostaining, CD106 is conceived to be a molecule that does not contribute to the cartilage differentiation potency of cells.

### <Example 6> Checking of effect of regenerating meniscus due to rat synovium-derived mesenchymal stem cells in which integrin β1 has been inhibited

The preparation of rat synovium-derived mesenchymal stem cells in which integrin β1 was inhibited was carried out as described in Example 2. A frozen stock was woken up, the culture was carried out for one week, and the recovered cells were suspended in a reaction solvent of PBS containing 2% FBS. 12 µg of Purified anti-mouse/rat integrin β1 Antibody per number of cells of 5 × 10⁶ cells was added thereto and reacted with cells for 1 hour under ice-cooling, and then the cells were recovered for transplantation (integrin β1-rSMSC). In addition, as a control treatment in which the inhibition of integrin β1 was not carried out, Purified Armenian Hamster IgG Isotype Ctrl was reacted with cells for 1 hour under ice-cooling, and then the cells were recovered for transplantation (IgG-rSMSC).

A LEW/CrlCrlj rat was used to prepare a meniscus injury model for evaluating the effect of regenerating the meniscus. In the method of transplanting meniscus injuries and mesenchymal stem cells, the skin of the knee joint part was incised under isoflurane anesthesia to expose the knee joint. The inner joint capsule under the patella was exposed, and a longitudinal incision was made with a scalpel to expose the cartilage in the distal end part of the femur. The medial meniscus was detached from the synovium to expose the medial meniscus, and about 2/3 of the whole thereof was excised. The patellar tendon and the synovium were sutured, and then the muscles were sutured to prepare a meniscus injury model. Thereafter, the treated animals were distributed to three groups, and then 5 × 10⁶ cells of the synovial stem cells (integrin β1-rSMSC) in which integrin β1 had been inhibited, 5 × 10⁶ cells of the synovial stem cells (IgG-rSMSC) which had been subjected to a control treatment in which the inhibition had not been carried out, and only the solvent were each administered into the joint capsule. On the day after cell administration, 12 µg of Purified anti-mouse/rat integrin β1 Antibody, Armenian Hamster IgG Isotype Ctrl per knee, or a solvent was administered into the joint capsule. After the treatment, all rats were returned to the cages and allowed to exercise, eat, and drink freely.

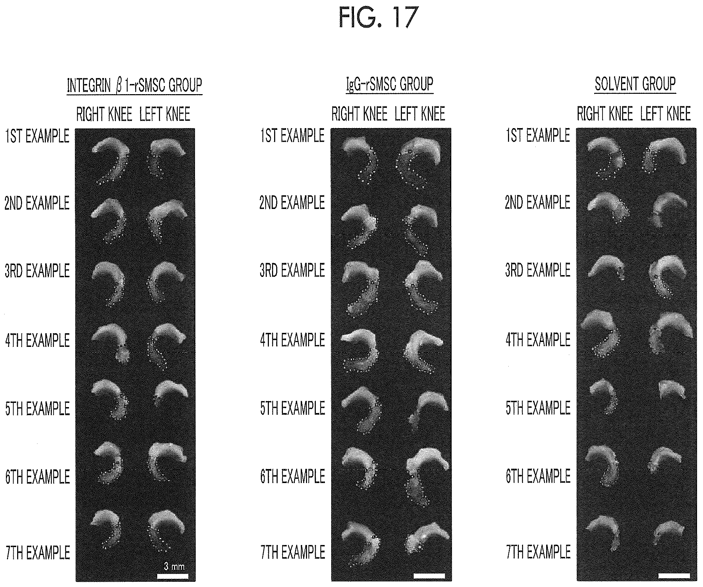

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 17. A regenerated portion was specified based on the difference in color tone and shape from the normal meniscus and surrounded by a broken line. In the solvent group which is a negative control, a large number of meniscuses had a shape in which the meniscus was observed up to the middle segment. In the IgG-rSMSC group which is a positive control, there are many cases in which the meniscus is regenerated up to the anterior segment, where the meniscus-regenerated portion is observed to be large. On the other hand, in the integrin β1-rSMSC group, which are cells that have undergone the molecular inhibition or molecular deletion, the meniscus-regenerated portion is observed to be small as compared with that in the positive control.

In order to quantitatively evaluate the gross appearance of the meniscus-regenerated portion in Fig. 17, the area of the meniscus-regenerated portion (inside the broken line) was calculated according to the following expression using ImageJ (version 1.52).

Area of meniscus-regenerated portion (mm²) = number of pixels in area of meniscus-regenerated portion/number of pixels per 1 mm²

The average value, standard deviation, and statistical analysis of the areas of regenerated portions of each group were all carried out by Microsoft Excel 2007 (Microsoft Corp.). For the statistical analysis, the positive control group and the molecular inhibition group, and the molecular inhibition group and the solvent group were subjected to two times of the Student's T-Test, and P values were respectively calculated. The Bonferroni correction was applied since the test was repeated, and a value obtained by multiplying the calculated P value by the number of times of test (2) was adopted. A significance level of 5% (α = 0.05) was regarded as having a difference, and the results are shown in Table 1.

With regard to the average area value of the meniscus-regenerated portions shown in Table 1, 2.1 mm² of the integrin β1-rSMSC group was significantly reduced with respect to 3.4 mm² of the IgG-rSMSC. On the other hand, the integrin β1-rSMSC group had a regenerated area, which was approximately the same as 1.7 mm² of the solvent group. From this, it was confirmed that the integrin β1 molecule in the synovial stem cells is an important molecule that contributes to the regeneration of the meniscus.

**[Table 1]**

| Table 1: Area of meniscus-regenerated portion (average value ± standard deviation) | |
|---|---|
| Group | Area (mm²) |
| Integrin β1-rSMSC | 2.1 ± 0.5* |
| IgG-rSMSC | 3.4 ± 1.0 |
| Solvent | 1.7 ± 0.9 |

| | |
|---|---|
| *P < 0.05 vs IgG-rSMSC group | |

### <Example 7> Checking of effect of regenerating meniscus due to rat synovium-derived mesenchymal stem cells in which PDGFRb has been inhibited

The preparation of rat synovium-derived mesenchymal stem cells in which PDGFRb was inhibited was carried out as described in Example 3. A frozen stock was woken up, the culture was carried out for one week, and the recovered cells were suspended in a reaction solvent of PBS containing 2% FBS. 12 µg of Anti-PDGF Receptor β Human Goat-Poly (R & D Systems Cat. No. AF385) per number of cells of 5 × 10⁶ cells was reacted with cells for 1 hour under ice-cooling, and then the cells were recovered for transplantation (PDGFRb-rSMSC). In addition, as a control treatment in which the inhibition of PDGFRb was not carried out, Normal Goat IgG Control (R & D Systems Cat. No. AB-108-C) was reacted with cells for 1 hour under ice-cooling, and then the cells were recovered for transplantation (IgG-rSMSC).

A meniscus injury model for evaluating the effect of regenerating the meniscus was prepared as described in Example 6. Thereafter, the treated animals were distributed to three groups, and then 5 × 10⁶ cells of the synovial stem cells (PDGFRb-rSMSC) in which PDGFRb had been inhibited, 5 × 10⁶ cells of the synovial stem cells (IgG-rSMSC) which had been subjected to a control treatment in which the inhibition had not been carried out, and only the solvent were each administered into the joint capsule. On the day after cell administration, 12 µg of Anti-PDGF Receptor β Human Goat-Poly, Normal Goat IgG Control per knee, or a solvent was administered into the joint capsule. After the treatment, all rats were returned to the cages and allowed to exercise, eat, and drink freely.

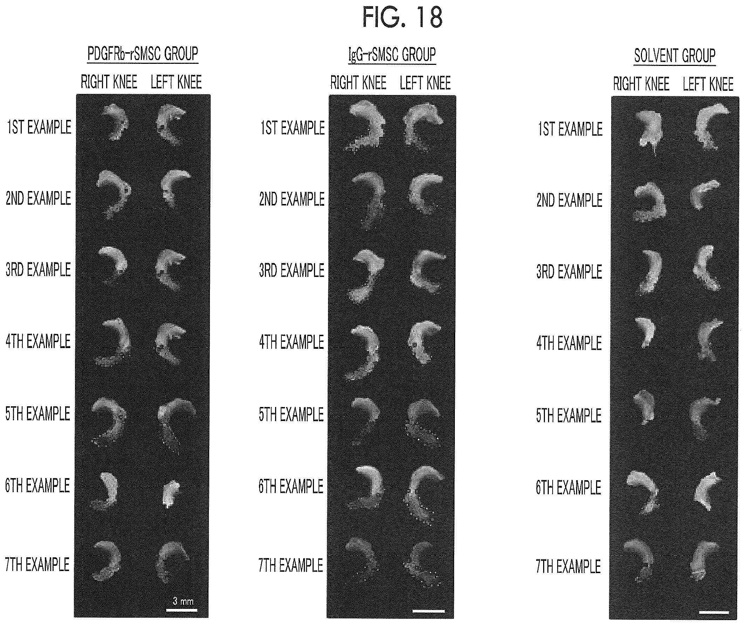

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 18. A regenerated portion was specified based on the difference in color tone and shape from the normal meniscus and surrounded by a broken line. In the solvent group which is a negative control, a large number of meniscuses had a shape in which the meniscus was observed up to the middle segment. In the IgG-rSMSC group which is a positive control, there are many cases in which the meniscus is regenerated up to the anterior segment, where the meniscus-regenerated portion is observed to be large. On the other hand, in the PDGFRb-rSMSC group, which are cells that have undergone the molecular inhibition or molecular deletion, the meniscus-regenerated portion is observed to be small as compared with that in the positive control.

In order to quantitatively evaluate the gross appearance of the meniscus-regenerated portion in Fig. 18, the area of the meniscus-regenerated portion (inside the broken line) was measured as described in Example 6. The results are shown in Table 2. With regard to the average area value of the meniscus-regenerated portions, 2.2 mm² of the PDGFRb-rSMSC group was significantly reduced with respect to 3.2 mm² of the IgG-rSMSC group. On the other hand, the average area value of the meniscus-regenerated portions of the PDGFRb-rSMSC group was significantly increased with respect to 1.3 mm² of the solvent group. From this result, it was confirmed that the PDGFRb molecule in the synovial stem cells is a molecule that contributes to the regeneration of the meniscus.

**[Table 2]**

| Table 2: Area of meniscus-regenerated portion (average value ± standard deviation) | |
|---|---|
| Group | Area (mm²) |
| PDGFRb-rSMSC | 2.2 ± 0.9*^{†} |
| IgG-rSMSC | 3.2 ± 0.7 |
| Solvent | 1.3 ± 1.2 |

| | |
|---|---|
| *P < 0.05 vs IgG-rSMSC group †P < 0.05 vs solvent group | |

### <Comparative Example 5> Checking of effect of regenerating meniscus due to rat synovium-derived mesenchymal stem cells in which CD44 has been inhibited

For the preparation of rat synovium-derived mesenchymal stem cells in which CD44 was inhibited, a frozen stock was woken up, the culture was carried out for one week, and the recovered cells were suspended in a reaction solvent of PBS containing 2% FBS. 12 µg of Anti-CD44 Rabbit IgG clone Hermes-1 (Absolute Antibody Cat. No. Ab00628-23.0) per number of cells of 5 × 10⁶ cells was reacted with cells for 30 minutes under ice-cooling, and then the cells were recovered for transplantation (CD44-rSMSC). In addition, as a control treatment in which the inhibition was not carried out, Rabbit IgG Isotype Control (Invitrogen Cat. No. 10500C) was reacted with cells for 1 hour under ice-cooling, and the cells were recovered for transplantation (IgG-rSMSC).

A meniscus injury model for evaluating the effect of regenerating the meniscus was prepared as described in Example 6. Thereafter, the treated animals were distributed to three groups, and then 5 × 10⁶ cells of the synovial stem cells (CD44-rSMSC) in which CD44 had been inhibited, 5 × 10⁶ cells of the synovial stem cells (IgG-rSMSC) which had been subjected to a control treatment in which the inhibition had not been carried out, and only the solvent were each administered into the joint capsule. On the day after cell administration, 12 µg of Anti-CD44 Rabbit IgG clone Hermes-1, Rabbit IgG Isotype Control per knee, or a solvent was administered into the joint capsule. After the treatment, all rats were returned to the cages and allowed to exercise, eat, and drink freely.

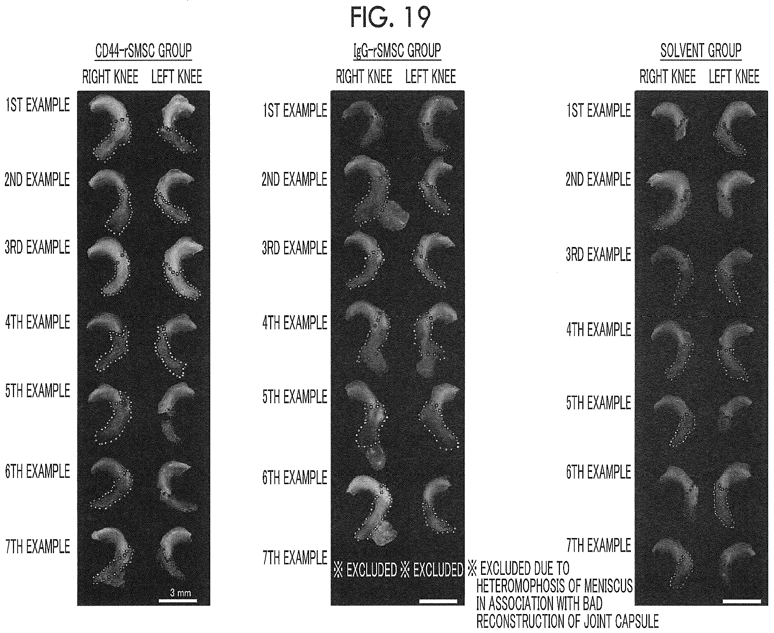

Four weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 19. A regenerated portion was specified based on the difference in color tone and shape from the normal meniscus and surrounded by a broken line. In the solvent group which is a negative control, a large number of meniscuses had a shape in which the meniscus was observed up to the middle segment. In the IgG-rSMSC group which is a positive control and the CD44-rSMSC group, there are many cases in which the meniscus is regenerated up to the anterior segment, where the meniscus-regenerated portion is observed to be large.

In order to quantitatively evaluate the gross appearance of the meniscus-regenerated portion in Fig. 19, the area of the meniscus-regenerated portion (inside the broken line) was measured as described in Example 6. The results are shown in Table 3. With regard to the average area value of the meniscus-regenerated portions, there was no significant difference between 3.4 mm² of the CD44-rSMSC group and 4.0 mm² of the IgG-rSMSC, which were approximately the same. On the other hand, the average area value of the meniscus-regenerated portions of the CD44-rSMSC group was significantly increased with respect to 2.7 mm² of the solvent group. From this, it is conceived that CD44 in the synovial stem cells is a molecule that does not contribute to the regeneration of the meniscus.

**[Table 3]**

| Table 3: Area of meniscus-regenerated portion (average value ± standard deviation) | |
|---|---|
| Group | Area (mm²) |
| CD44-rSMSC | 3.4 ± 0.9† |
| IgG-rSMSC | 4.0 ± 1.0 |
| Solvent | 2.7 ± 0.7 |

| | |
|---|---|
| †P < 0.05 vs solvent group | |

### <Example 8> Checking of effect of regenerating meniscus due to rat synovium-derived mesenchymal stem cells from which Col2A1 (Col2A1KO-rSMSC) has been deleted.

Rat synovium-derived mesenchymal stem cells from which Col2A1 had been deleted were adjusted for transplantation by expansion culture after confirming the deletion of Col2A1, as described in Example 4 (Col2A1KO-rSMSC). The wild-type sequence of Col2A1 as a positive control was confirmed, and then the cells were adjusted for transplantation by expansion culture (Col2A1WT-rSMSC).

A meniscus injury model for evaluating the effect of regenerating the meniscus was prepared as described in Example 6. Thereafter, the treated animals were distributed to three groups, and then 5 × 10⁶ cells of each of the Col2A1KO-rSMSC and the Col2A1WT-rSMSC and only the solvent were each administered into the joint capsule.

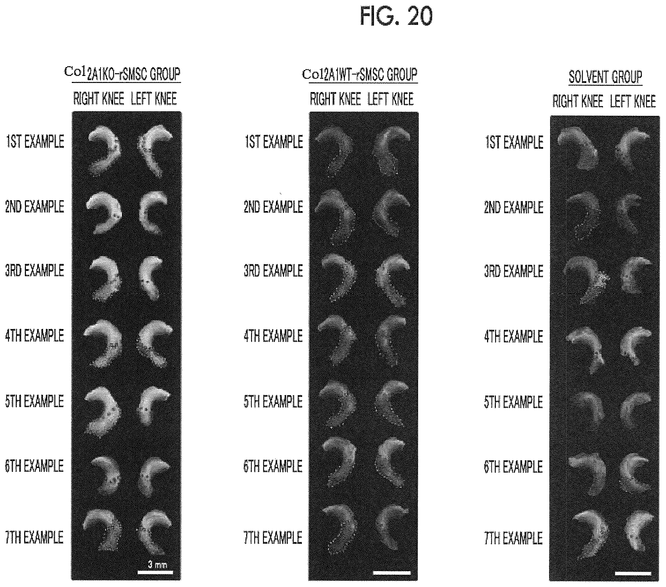

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 20. A regenerated portion was specified based on the difference in color tone and shape from the normal meniscus and surrounded by a broken line. In the solvent group which is a negative control, a large number of meniscuses had a shape in which the meniscus was observed up to the middle segment. In the Col2A1WT-rSMSC group which is a positive control, there are many cases in which the meniscus is regenerated up to the anterior segment, where the meniscus-regenerated portion is observed to be large. On the other hand, in the Col2A1KO-rSMSC group, the meniscus-regenerated portion is observed to be small as compared with that in the positive control.

In order to quantitatively evaluate the gross appearance of the meniscus-regenerated portion in Fig. 20, the area of the meniscus-regenerated portion (inside the broken line) was measured as described in Example 6. The results are shown in Table 4. With regard to the average area value of the meniscus-regenerated portions, the Col2A1WT-rSMSC was significantly increased to 3.8 mm², as compared with 2.9 mm² of the Col2A1KO-rSMSC group. On the other hand, the average area value of the meniscus-regenerated portions of the Col2A1KO-rSMSC group was significantly increased as compared with 2.1 mm² of the solvent group. From this result, it was confirmed that the Col2A1 molecule in the synovial stem cells is a molecule that contributes to the regeneration of the meniscus.

**[Table 4]**

| Table 4: Area of meniscus-regenerated portion (average value ± standard deviation) | |
|---|---|
| Group | Area (mm²) |
| Col2A1KO-rSMSC | 2.9 ± 0.8*† |
| Col2A1WT-rSMSC | 3.8 ± 0.6 |
| Solvent | 2.1 ± 1.0 |

| | |
|---|---|
| *P < 0.05 vs IgG-rSMSC group †P < 0.05 vs solvent group | |

<Comparative Example 6> Checking of effect of regenerating meniscus due to rat synovium-derived mesenchymal stem cells from which CD120a (CD120aKO-rSMSC) has been deleted.

Rat synovium-derived mesenchymal stem cells from which CD120a had been deleted were adjusted for transplantation by expansion culture after confirming the deletion of CD120a, as described in Comparative Example 1 (CD120aKO-rSMSC). The wild-type sequence of CD120a as a positive control was confirmed, and then the cells were adjusted for transplantation by expansion culture (CD120aWT-rSMSC).

A meniscus injury model for evaluating the effect of regenerating the meniscus was prepared as described in Example 6. Thereafter, the treated animals were distributed to three groups, and then 5 × 10⁶ cells of each of the CD120aKO-rSMSC and the CD120aWT-rSMSC and only the solvent were each administered into the joint capsule.

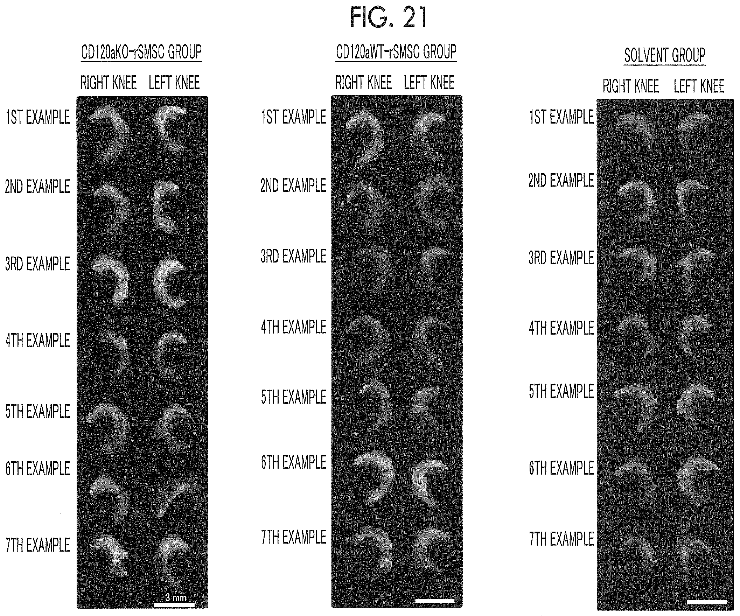

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 21. A regenerated portion was specified based on the difference in color tone and shape from the normal meniscus and surrounded by a broken line. In the solvent group which is a negative control, a large number of meniscuses had a shape in which the meniscus was observed up to the middle segment. On the other hand, in the CD120aWT-rSMSC group which is a positive control and in the CD120aKO-rSMSC group, there are many cases in which the meniscus is regenerated up to the anterior segment, where the meniscus-regenerated portion is observed to be large as compared with that in the solvent group.

In order to quantitatively evaluate the gross appearance of the meniscus-regenerated portion in Fig. 21, the area of the meniscus-regenerated portion (inside the broken line) was measured as described in Example 6. The results are shown in Table 5. With regard to the average area value of the meniscus-regenerated portions, the CD120aWT-rSMSC was 3.3 mm² as compared with 3.1 mm² of the CD120aKO-rSMSC group, and thus no significant difference was observed. On the other hand, the average area value of the meniscus-regenerated portions of the CD120aKO-rSMSC group was significantly increased as compared with 1.8 mm² of the solvent group. From this, it is conceived that CD120a in the synovial stem cells is a molecule that does not contribute to the regeneration of the meniscus.

**[Table 5]**

| Table 5: Area of meniscus-regenerated portion (average value ± standard deviation) | |
|---|---|
| Group | Area (mm²) |
| CD120aKO-rSMSC | 3.1 ± 0.7† |
| CD120aWT-rSMSC | 3.3 ± 1.6 |
| Solvent | 1.8 ± 0.5 |

| | |
|---|---|
| †P < 0.05 vs solvent group | |

<Comparative Example 7> Checking of effect of regenerating meniscus due to rat synovium-derived mesenchymal stem cells from which CD106 (CD106KO-rSMSC) has been deleted.

Rat synovium-derived mesenchymal stem cells from which CD106 had been deleted were adjusted for transplantation by expansion culture after confirming the deletion of CD106, as described in Comparative Example 1 (CD106KO-rSMSC). The wild-type sequence of CD106 as a positive control was confirmed, and then the cells were adjusted for transplantation by expansion culture (CD106WT-rSMSC).

A meniscus injury model for evaluating the effect of regenerating the meniscus was prepared as described in Example 6. Thereafter, the treated animals were distributed to three groups, and then 5 × 10⁶ cells of each of the CD106KO-rSMSC and the CD106WT-rSMSC and only the solvent were each administered into the joint capsule.

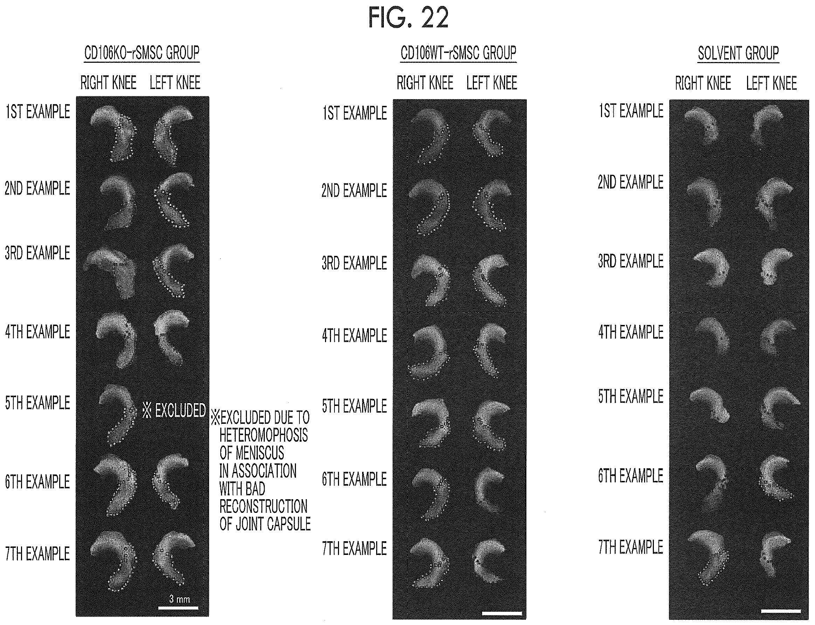

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 22. A regenerated portion was specified based on the difference in color tone and shape from the normal meniscus and surrounded by a broken line. In the solvent group which is a negative control, a large number of meniscuses had a shape in which the meniscus was observed up to the middle segment. On the other hand, in the CD106WT-rSMSC group which is a positive control and in the CD106KO-rSMSC group, there are many cases in which the meniscus is regenerated up to the anterior segment, where the meniscus-regenerated portion is observed to be large as compared with that in the solvent group.

In order to quantitatively evaluate the gross appearance of the meniscus-regenerated portion in Fig. 22, the area of the meniscus-regenerated portion (inside the broken line) was measured as described in Example 6. The results are shown in Table 6. With regard to the average area value of the meniscus-regenerated portions, the CD106WT-rSMSC was 3.3 mm² as compared with 3.7 mm² of the CD106KO-rSMSC group, and thus no significant difference was observed. On the other hand, the average area value of the meniscus-regenerated portions of the CD106KO-rSMSC group was significantly increased as compared with 1.7 mm² of the solvent group. From this, it is conceived that CD106 in the synovial stem cells is a molecule that does not contribute to the regeneration of the meniscus.

**[Table 6]**

| Table 6: Area of meniscus-regenerated portion (average value ± standard deviation) | |
|---|---|
| Group | Area (mm²) |
| CD106KO-rSMSC | 3.7 ± 0.7† |
| CD106WT-rSMSC | 3.3 ± 0.7 |
| Solvent | 1.7 ± 0.7 |

| | |
|---|---|
| †P < 0.05 vs solvent group | |

### <Example 9> Rat synovium-derived mesenchymal stem cell establishment step, and difference in treatment time and expression rates of integrin β1 and PDGFRb during cell recovery

In the same manner as in Example 1, cells were isolated from the rat synovium and cultured for 8 days to obtain rat synovium-derived mesenchymal stem cells. The culture medium in the flask was discarded, and washing was carried out twice with PBS. Then, TrypLE Express (Gibco Cat. No. 12604-013) was added thereto, and the cells were allowed to stand in an incubator of 37°C for 5, 30, 60, or 120 minutes and then detached and recovered.

10⁶ cells of the cells recovered under each condition were suspended in 500 µL of PBS. For staining of dead cells, a LIVE/DEAD Fixable Aqua Dead Cell Stain Kit (Invitrogen Cat. No. L34957) was added to 0.5 µL of the cell suspension, and incubation was carried out at room temperature for 30 minutes. After centrifugation, the supernatant was discarded, 1 mL of a FACS buffer (PBS containing EDTA 2Na of a final concentration of 2 mmol/L and bovine serum albumin of a final concentration of 1%) was added thereto, and the cells were suspended. In order to measure the expression rate of integrin β1, 5 µL of PE anti-mouse/rat integrin β1 Antibody (Biolegend Cat. No. 102207) or PE Armenian Hamster IgG Isotype Ctrl Antibody (Biolegend Cat. No. 400907) was added thereto, and a reaction was carried out at 4°C for 30 minutes. Thereafter, centrifugation was carried out to discard the supernatant, the suspension was carried out in 1 mL of a FACS buffer, centrifugation was carried out again, the supernatant was discarded, and then the cells were suspended in 500 µL of a FACS buffer and subjected to a measurement. In order to measure the expression rate of PDGFRb, 5 µL of Anti-PDGF Receptor β, Human, Goat-Poly (R & D Systems Cat. No. AF385), or Normal Goat IgG Control (R & D Systems Cat. No. AB-108-C) was added thereto, and a reaction was carried out at 4°C for 30 minutes. Thereafter, centrifugation was carried out to discard the supernatant, and the cells were suspended in 1 mL of a FACS buffer. Further, 1 µL of Donkey anti-Goat IgG (H + L) Cross-Adsorbed Secondary Antibody, FITC (Invitrogen Cat. No. A16006) was added thereto, and a reaction was carried out at 4°C for 30 minutes. Thereafter, centrifugation was carried out to discard the supernatant, and the suspension was carried out in 1 mL of a FACS buffer, centrifugation was carried out again, the supernatant was discarded, and then the cells were suspended in 500 µL of a FACS buffer and subjected to a measurement with a flow cytometer (Attune NxT, Auto Focusing Cytometer, model: AFC2, Invitrogen), whereby the expression rates of integrin β1 and PDGFRb were measured.

Table 7 shows the expression rates of integrin β1 and PDGFRb depending on the difference in the detachment treatment time for the synovium-derived mesenchymal stem cells. Even in a case where the detachment treatment time was extended from a typical time of 5 minutes to 120 minutes, the expression rate of integrin β1 was maintained at 90% or more. On the other hand, the expression rate of PDGFRb was decreased in a time-dependent manner, and it was 91.7% at 5 minutes, 76.9% at 30 minutes, 63.3% at 60 minutes, and 32.8% at 120 minutes. Since PDGFRb is a molecule necessary for regenerating the meniscus in the synovial stem cells as shown in Example 7, and the effect of regenerating the meniscus can be further expected in a case where the expression rate of PDGFRb is larger than half of 100% in the cell detachment treatment time, the treatment time is preferably 60 minutes or less, and the required expression rate of this molecule can be set to 60% or more.

**[Table 7]**

| Table 7: Detachment treatment time and expression rates of integrin β1 and PDGFRb during cell recovery | | | | |
|---|---|---|---|---|
| Expression rate | Treatment time | | | |
| | 5 minutes | 30 minutes | 60 minutes | 120 minutes |
| Integrin β1 | 98.7% | 99.7% | 96.1% | 97.8% |
| PDGFRb | 91.6% | 76.9% | 63.3% | 32.8% |

### <Example 10> Number of days of culture and expression rate of integrin β1 during cell recovery in establishment of rat synovium-derived mesenchymal stem cell

In the same manner as in Example 1, cells were isolated from the rat synovium, and 7.5 × 10⁴ cells were seeded in a flask having an area of 75 cm² and cultured for 8, 21, or 28 days to obtain rat synovium-derived mesenchymal stem cells. The culture medium in the flask was discarded, and washing was carried out twice with PBS. Then, TrypLE Express (Gibco Cat. No. 12604-013) was added thereto, and the cells were allowed to stand in an incubator of 37°C for 120 minutes and then detached and recovered. Thereafter, in the same manner as in Example 9, the dead cells and the integrin β1 were subjected to a staining operation and then subjected to a measurement.

Table 8 shows the expression rate of integrin β1 depending on the difference in the number of days of culture for the synovium-derived mesenchymal stem cells. The expression rate of integrin β1 of the synovium-derived mesenchymal stem cells was decreased in a manner dependent on the number of days of culture, and it was 97.8% after 8 days, 62.1% after 21 days, and 56.1% after 28 days. Since integrin β1 is a molecule necessary for regenerating the meniscus in the synovial stem cells as shown in Example 6, the cells are desirable to be such that the integrin β1-positive rate is larger than half of 100%. In addition, since the expression rate of the molecule required for regenerating the meniscus can be set to 60% or more as shown in Example 7, the number of days of culture is desirable to be 21 days or less.

**[Table 8]**

| Table 8: Culture time and expression rate of integrin β1 after cell isolation | | | |
|---|---|---|---|
| Expression rate | Number of days of culture | | |
| | 8 days | 21 days | 28 days |
| Integrin β1 | 97.8% | 62.1% | 56.1% |

### <Example 11>

The positive rates of integrin β1 and PDGFRb, which are protein molecules essential for drug efficacy, were also measured by flow cytometry in human synovium-derived stem cells (Cryopreserved Synoviocytes, Normal, P1, model number: CDD-H-2910-N, lot: ST1414, ST1420, ST1434, ST1462) purchased from Articular Engineering, LLC. As a measurement device, Attune NxT, Auto Focusing Cytometer (model: AFC2, Invitrogen), was used. As an antibody, each of integrin β1 (APC Mouse Anti-Human CD29, Cat: 559883) and PDGFRb (Anti-PDGF Receptor β, Human, Goat-Poly, Cat: AF385) was used.

As a result, the surface antigen positive rate of integrin β1 was 99.4%, 99.6%, 99.6%, and 99.5% for each lot. In addition, it was found that the surface antigen positive rate of PDGFRb was 92.2%, 90.7%, 95.2%, and 85.9% for each lot. From this, it was found that in a case where this human synovium-derived stem cell has a therapeutic effect as a therapeutic agent for arthrosis, it is reasonable to carry out management such that a standard value for integrin β1 is 90% or more, and a standard value for PDGFRb is 80%.

As described above, the expressions of integrin β1 and PDGFRb could be confirmed not only in the rat synovium-derived stem cells in Examples 9 and 10 but also in the human synovium-derived stem cells, and thus these have been shown to be capable of being set as the effectiveness quality management items for cells.

### <Example 12> Checking of effect of regenerating meniscus due to rat synovium-derived stem cells in which FGFR3 has been inhibited

For the preparation of rat synovium-derived stem cells in which FGFR3 was inhibited, a frozen stock of the rat synovium-derived stem cells prepared in Example 1 was woken up and cultured at a CO₂ concentration of 5% and 37°C for one week using αMEM no nucleosides to which Fetal Bovine Serum was added to a final concentration of 20%, L-glutamine 200 mmol/L was added to a final concentration of 1%, and Antibiotic-Antimycotic (100×) was added to a final concentration of 1%, and the recovered cells were suspended in a reaction solvent of PBS containing 2% FBS. 100 µg of FGFR3 Polyclonal Antibody (Invitrogen Cat. No. PA5-34574) per number of cells of 5 × 10⁶ cells was added thereto, and the cells were recovered after being reacted for 1 hour under ice-cooling (FGFR3-rSMSC). As a control treatment in which FGFR3 was not inhibited, rabbit IgG Isotype Control (Thermo Fisher Scientific, Inc. Cat. No. 10500C) was reacted for 1 hour under ice-cooling, and then the cells were recovered (IgG-rSMSC).

A LEW/CrlCrlj rat was used to prepare a meniscus injury model for evaluating the effect of regenerating the meniscus. In the method of transplanting meniscus injuries and mesenchymal stem cells, the skin of the knee joint part was incised under isoflurane anesthesia to expose the knee joint. The inner joint capsule under the patella was exposed, and a longitudinal incision was made with a scalpel to expose the cartilage in the distal end part of the femur. The medial meniscus was detached from the synovium to expose the medial meniscus, and about 2/3 thereof from the anterior end was excised. The patellar tendon and the synovium were sutured, and then the muscles were sutured to prepare a meniscus injury model. Thereafter, the treated animals were distributed to three groups, and then 5 × 10⁶ cells of the synovial stem cells (FGFR3-rSMSC) in which FGFR3 had been inhibited, 5 × 10⁶ cells of the synovial stem cells (IgG-rSMSC) which had been subjected to a control treatment in which the inhibition had not been carried out, and only the solvent were each administered into the joint capsule. After the treatment, all rats were returned to the cages and allowed to exercise, eat, and drink freely.

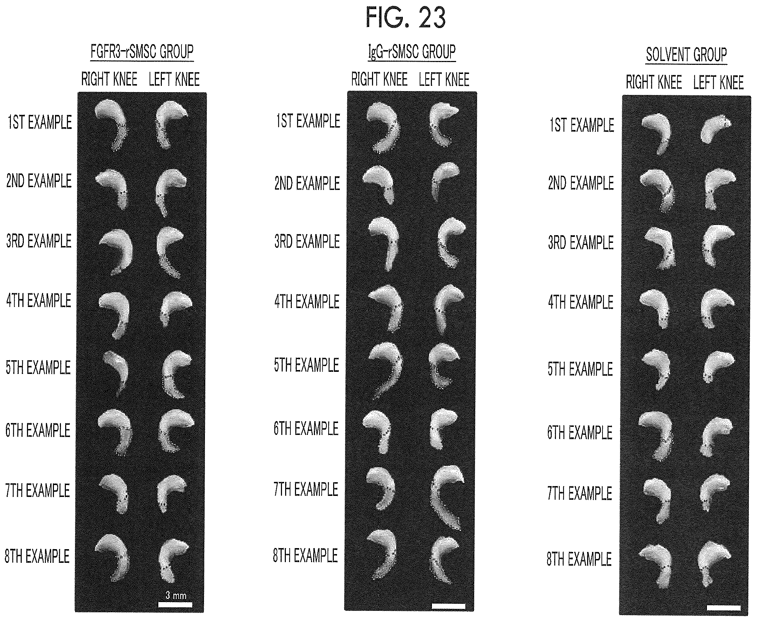

Three weeks after the treatment, the animals were euthanized by cutting the descending aorta under isoflurane anesthesia. Then, the meniscus was exposed from the knee joint, and a photographic image was captured. The images of the excised medial meniscus of both knee joints are shown in Fig. 23. A regenerated portion was specified based on the difference in color tone and shape from the normal meniscus and surrounded by a broken line. In the solvent group which is a negative control, a large number of meniscuses had a shape in which the meniscus was observed up to the middle segment. In the IgG-rSMSC group which is a positive control, there are many cases in which the meniscus is regenerated up to the anterior segment, where the meniscus-regenerated portion is observed to be large. On the other hand, in the FGFR3-rSMSC group, which are cells that have undergone the molecular inhibition or molecular deletion, the meniscus-regenerated portion is observed to be small as compared with that in the positive control.

In order to quantitatively evaluate the gross appearance of the meniscus-regenerated portion in Fig. 23, the area of the meniscus-regenerated portion (inside the broken line) was calculated according to the following expression using ImageJ (version 1.52).

Area of meniscus-regenerated portion (mm²) = number of pixels in area of meniscus-regenerated portion/number of pixels per 1 mm²

The average value, standard deviation, and statistical analysis of the areas of regenerated portions of each group were all carried out by Microsoft Excel 2007 (Microsoft Corp.). For the statistical analysis, the positive control group and the molecular inhibition group, and the molecular inhibition group and the solvent group were subjected to two times of the Student's T-Test, and P values were respectively calculated. In addition, A significance level of 5% (α = 0.05) and a significance level of 2.5% (α = 0.025) obtained by being divided by the number of times of test (2) according to the Bonferroni correction were regarded as having a difference, and the results are shown in Table 9.

With regard to the average area value of the meniscus-regenerated portions shown in Table 9, 1.2 mm² of the FGFR3-rSMSC group was significantly reduced with respect to 1.9 mm² of the IgG-rSMSC group. On the other hand, the FGFR3-rSMSC group had a regenerated area which was approximately the same as 1.0 mm² of the solvent group. From this result, it was confirmed that the FGFR3 molecule in the synovial stem cells is an important molecule that contributes to the regeneration of the meniscus.

**[Table 9]**

| Table 9: Area of meniscus-regenerated portion (average value ± standard deviation) | |
|---|---|
| Group | Area (mm²) |
| FGFR3-rSMSC | 1.2 ± 0.5*† |
| IgG-rSMSC | 1.9 ± 0.6 |
| Solvent | 1.0 ± 0.5 |

| | |
|---|---|
| *P < 0.025 vs IgG-rSMSC group †P < 0.025 vs solvent group | |

### [Sequence list]

International application based on the International Patent Cooperation Treaty 21F00805WlJP22032502_9.xml

## Claims

1. A therapeutic agent for arthrosis, comprising:
synovium-derived mesenchymal stem cells having any one or more kinds of surface antigens of integrin β1 or platelet-derived growth factor receptor β.

2. The therapeutic agent for arthrosis according to claim 1,
wherein the synovium-derived mesenchymal stem cells have surface antigens of both the integrin β1 and the platelet-derived growth factor receptor β.

3. The therapeutic agent for arthrosis according to claim 1 or 2,
wherein the synovium-derived mesenchymal stem cells have a gene encoding a type II collagen α1 chain and produce the type II collagen α1 chain after being transplantation.

4. The therapeutic agent for arthrosis according to claim 1 or 2,
wherein the synovium-derived mesenchymal stem cells have a surface antigen of FGFR3.

5. The therapeutic agent for arthrosis according to claim 1 or 2,
wherein a ratio of the synovium-derived mesenchymal stem cells having any one or more kinds of surface antigens of integrin β1 or platelet-derived growth factor receptor β to all cells contained in the therapeutic agent for arthrosis is 30% or more.

6. A production method for the therapeutic agent for arthrosis according to claim 1, comprising:
a step A of treating a synovial tissue with an enzyme;
a step B of washing an enzyme-treated mixture;
a step C of culturing synovium-derived mesenchymal stem cells, which are contained in a washed mixture, on a base material; and
a step D of separating cultured synovium-derived mesenchymal stem cells from the base material.

7. The method according to claim 6,
wherein the step B is a step of washing the enzyme-treated mixture until a residual enzyme concentration in a supernatant is 0.5 ng/mL or less.

8. The method according to claim 6 or 7,
wherein in the step C, a period during which the synovium-derived mesenchymal stem cells are cultured is 28 days or less.

9. The method according to claim 6 or 7,
wherein in the step D, the mesenchymal stem cells are separated by allowing a cell stripper to act on the mesenchymal stem cells for a time within 120 minutes.

10. The method according to claim 6 or 7, further comprising:
a step of sorting out synovium-derived mesenchymal stem cells having any one or more kinds of surface antigens of integrin β1 or platelet-derived growth factor receptor β.
